# EUROPEAN PATENT APPLICATION

(11) **EP 3 536 803 A1**
(43) Date of publication of application: **11.09.2019**
(21) Application number: 17867710.0
(22) Date of filing: 02.11.2017
(51) Int. Cl.: C12Q 1/68, C12N 15/10, C12N 15/115, A61K 31/7088, G06F 19/20

(54) **QUANTITATIVE CLUSTER ANALYSIS METHOD OF TARGET PROTEIN BY USING NEXT-GENERATION SEQUENCING AND USE THEREOF**

(30) Priority: 02.11.2016 KR 20160144860
(71) Applicant: Biois Co., Ltd., Seoul 08390 (KR)
(72) Inventor: KIM, Sung Chun, Seodaemun-gu Seoul 03733 (KR)
(74) Representative: Betten & Resch
(86) International application number: PCT/KR2017/012323
(87) International publication number: WO 2018/084594

(57) **Abstract**

Disclosed is a method of quantitatively analyzing a target protein population in a sample to be analyzed, the method including (a) treating a sample to be analyzed with an aptamer library specific to a target protein population present in the sample so as to form complexes between target proteins and aptamers binding specifically thereto, thereby forming a target protein-aptamer complex population, (b) isolating the complex population from unbound aptamers, and (c) analyzing the sequence of each aptamer of the complex population through a next-generation sequencing process so as to quantify each aptamer of the complex population, thereby quantifying each target protein in the complex population. The method of the present invention can be very useful in collectively quantifying proteins in an analytical sample.

## Description

### Technical Field

The present invention relates to a method of quantitatively analyzing a target protein population using a next-generation sequencing process and the use thereof.

### Background Art

Techniques for analyzing multiple proteins that constitute a sample, techniques for producing protein profiles, which are comprehensive information on the quantitative and qualitative status of proteins contained in a sample, and techniques for identifying target molecules have been widely developed owing to the advancement of physics, biochemistry, and bioinformatics. However, there is a great need for efficient new methods and devices due to problems related to the use and maintenance costs of existing methods or devices, ease of use, accuracy, sensitivity, assay time, and process automation.

Recently, 2-D gel electrophoresis and mass spectrometry have been developed, and enable the measurement of small amounts of plasma components. However, it is required to carry out a labor-intensive preliminary classification protocol of removing the plasma/serum of proteins present at high concentrations (Anderson, Proteomics (3005); Anderson and Anderson, Electrophoresis (1991); Gygi and Aebersold, Curr. Opin. Chem. Biol. (3000); Liotta, et al., JAMA (3001); Yates, Trends Genet. (3000); and Adkin, et al., Mol. Cell Proteomics (3002)). Furthermore, these assays are time-consuming and costly due to the requirement to purchase and manage essential devices to perform these methods.

Proteomes of biosamples, for example, plasma proteomes, are very promising as convenient specimens for disease diagnosis and therapeutic monitoring, but existing assays and techniques have drawbacks, including sensitivity limitations, time and efficiency limitations, and related transitional costs. Furthermore, existing assays and techniques do not sufficiently utilize biosamples as raw materials for biomarkers. For example, both electrophoresis and mass spectrometry are capable of separating plasma proteins based on protein sizes and charges, but assays and techniques based on the other properties of proteins are lacking. There is thus demand in the art for methods of obtaining and utilizing a proteomic profile of a sample, and many attempts have been made to overcome the disadvantages of the existing techniques mentioned above.

Aptamers are ligands, which are single-stranded nucleic acids specific to target compounds or proteins and having high specific binding to target proteins. As a method of producing an aptamer, a "SELEX" (Systematic Evolution of Ligands by Exponential Enrichment) method is widely used. The SELEX method pertains to the in-vitro evolution of nucleic acid proteins having high specific binding to target molecules, as disclosed in U.S. Patent Application No. 07/536,428, filed June 11, 1990 (now abandoned), U.S. Patent No. 5,475,096 (Title: "Nucleic acid ligands") and U.S. Patent No. 5,270,163 (Title: "Nucleic acid ligands").

The SELEX method is based on the fact that nucleic acids possess a high ability to form a variety of two-dimensional and three-dimensional structures and sufficient chemical versatility in monomers to act as a ligand (i.e. forming a specific binding pair) for any chemical compound (either a monomer or a polymer). Proteins of any size or composition may be used as target molecules. Although aptamers have been extensively studied as very useful ligands, a typical aptamer selection process is limited because it is applied to known proteins or substances.

The present inventors have proposed processes of selecting single-stranded nucleic acids (molecule-binding nucleic acids) that bind to proteins in a composite sample containing unknown proteins or known proteins and quantifying target molecules that bind to the molecule-binding nucleic acids. Particularly, as disclosed in Reverse-SELEX for producing proteomic profiles (Korean Patent No. 10-0670799), Molecule-binding nucleic acid-based biochips (Korean Patent No. 10-0464225), and Biological meaning analysis technology using molecule-binding nucleic acid-based biochips (Korean Patent No. 10-0924048), methods of producing proteomic profiles using single-stranded nucleic acids and of selecting molecule-binding nucleic acids bound to multiple proteins constituting biosamples have been devised.

The present invention discloses a method of quantitatively analyzing a protein population in a sample using a next-generation sequencing (NGS) technique.

### Disclosure

### Technical Problem

An objective of the present invention is to provide a method of quantitatively analyzing a target protein population in a sample to be analyzed.

Another objective of the present invention is to provide a method of selecting a biomarker candidate protein by quantifying and comparing target protein populations in two or more samples to be analyzed.

Still another objective of the present invention is to provide a method of simultaneously analyzing target proteins and target nucleic acids in a sample to be analyzed.

Other specific objectives of the present invention will be set forth below.

### Technical Solution

An aspect of the present invention provides a method of quantitatively analyzing a target protein population in a sample to be analyzed, the method comprising: (a) treating a sample to be analyzed with an aptamer library specific to a target protein population present in the sample so as to form complexes between target proteins and aptamers binding specifically thereto, thereby forming a target protein-aptamer complex population, (b) isolating the complex population from unbound aptamers, and (c) analyzing the sequence of each aptamer of the complex population through a next-generation sequencing (NGS) process so as to quantify each aptamer of the complex population, thereby quantifying each target protein in the complex population.

In the method of the present invention, the aptamer library may be obtained by (i) preparing an aptamer pool having a random sequence to thus have potential binding capacity to various proteins, (ii) reacting the aptamer pool with the target protein population of the same sample as in step (a) so as to induce specific binding between aptamers and target proteins to thereby form a complex population, (iii) isolating the complex population by excluding unbound aptamers, and (iv) amplifying aptamers of the complex population.

Also in the method of the present invention, each aptamer of the aptamer library may have 5' and 3' regions comprising conserved regions of known sequences and a middle region therebetween comprising a variable region of any random sequence.

Also in the method of the present invention, the sample to be analyzed may be a processed sample obtained by removing a protein present in a large amount from the sample.

Also in the method of the present invention, step (c) may be performed by preparing a double-stranded DNA population from aptamers of the complex population and analyzing the double-stranded DNA population through a next-generation sequencing process. Here, each aptamer of the aptamer library may have 5' and 3' regions comprising conserved regions of known sequences and a middle region therebetween comprising a variable region of any random sequence, whereby the double-stranded DNA population may be prepared using a set of a forward primer and a reverse primer.

Also in the method of the present invention, the sample to be analyzed before treatment with the aptamer library in step (a) may be added with two or more external standard proteins having different quantification values (i.e. concentrations) that are absent in the sample, and the aptamer library in step (a) may use an aptamer library further including aptamers for the external standard proteins, whereby, in step (c), results of quantifying the aptamers for the external standard proteins may be obtained, in addition to results of quantifying the aptamers for the target proteins, and aptamer quantification results for the external standard proteins and aptamer quantification results for the target proteins are compared, thereby quantifying the target proteins.

Also in the method of the present invention, the aptamers may be single-stranded DNA or single-stranded RNA.

Also in the method of the present invention, the target protein population may be a population of unknown proteins, a population of known proteins, or a mixed population of unknown proteins and known proteins.

Also in the method of the present invention, when a certain protein of the target protein population is an unknown protein, isolating and identifying the unknown protein using an aptamer specific to the unknown protein that is contained in the aptamer library may be further performed.

Also in the method of the present invention, the quantifying in step (c) may be performed by counting the number of reads of the same sequence for the aptamers, counting the number of sequences that may be considered to be the same as the reads taking into account an error frequency of the next-generation sequencing process, and summing the number of reads and the number of sequences so that the target proteins are quantified based on the summed values. Here, the number of reads and the like may be counted by comparing a reference sequence, which is a known sequence for each aptamer obtained by analyzing the sequence of each aptamer of the aptamer library, with the sequence analysis result of each aptamer of the complex population.

Another aspect of the present invention provides a method of selecting a candidate protein as a biomarker, the method comprising: (a) treating a sample to be analyzed with an aptamer library specific to a target protein population present in the sample so as to form complexes between target proteins and aptamers binding specifically thereto, thereby forming a target protein-aptamer complex population, (b) isolating the complex population from unbound aptamers, and (c) analyzing the sequence of each aptamer of the complex population so as to quantify each aptamer of the complex population, thereby quantifying each target protein in the complex population, wherein the method further comprises: (i) performing steps (a) to (c) for an additional sample to be analyzed different from the sample to be analyzed, and (ii) determining one or more target proteins having different quantification results by comparing target protein quantification results obtained through step (c) between the two samples to be analyzed.

In the method of the present invention, the aptamer library may use the same aptamer library for the two samples to be analyzed.

Also in the method of the present invention, the same aptamer library for the two samples to be analyzed may be used, and the aptamer library may be obtained by (i) preparing an aptamer pool having a random sequence to thus have potential binding capacity to various proteins, (ii) reacting the aptamer pool with the target protein population of any one of the two samples to be analyzed so as to induce specific binding between aptamers and target proteins to thereby form a complex population, (iii) isolating the complex population by excluding unbound aptamers, and (iv) amplifying aptamers of the complex population.

Also in the method of the present invention, each aptamer of the aptamer library may have 5' and 3' regions comprising conserved regions of known sequences and a middle region therebetween comprising a variable region of any random sequence.

Also in the method of the present invention, each of the two samples to be analyzed may be a processed sample obtained by removing a protein present in a large amount from the sample.

Also in the method of the present invention, step (c) may be performed by preparing a double-stranded DNA population from the aptamers of the complex population and analyzing the double-stranded DNA population through a next-generation sequencing process. Here, each aptamer of the aptamer library may have 5' and 3' regions comprising conserved regions of known sequences and a middle region therebetween comprising a variable region of any random sequence, whereby the double-stranded DNA population may be prepared using a set of a forward primer and a reverse primer.

Also in the method of the present invention, the sample to be analyzed before treatment with the aptamer library in step (a) may be added with two or more external standard proteins having different quantification values that are absent in the sample, and the aptamer library in step (a) may use an aptamer library further including aptamers for the external standard proteins, whereby, in step (c), results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins; the additional sample to be analyzed before treatment with the aptamer library in step (i) may be added with two or more external standard proteins having different quantification values that are absent in the sample, and the aptamer library may use an aptamer library further including aptamers for the external standard proteins, whereby results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins, step (ii) may be performed by comparing target protein quantification results of the two samples to be analyzed, and the external standard proteins added to the two samples to be analyzed are preferably the same as each other.

Also in the method of the present invention, the aptamers are preferably single-stranded DNA or single-stranded RNA.

Also in the method of the present invention, the target protein population may be a population of unknown proteins, a population of known proteins, or a mixed population of unknown proteins and known proteins.

Also in the method of the present invention, when a certain protein of the target protein population is an unknown protein, isolating and identifying the unknown protein using an aptamer specific to the unknown protein that is contained in the aptamer library may be further performed.

Also in the method of the present invention, the quantifying in step (c) and the quantifying in step (i) may be performed by counting the number of reads of the same sequence for the aptamers, counting the number of sequences that may be considered to be the same as the reads taking into account an error frequency of the next-generation sequencing process, and summing the number of reads and the number of sequences so that the target proteins are quantified based on the summed values. As such, a reference sequence which is already determined may be used for counting the number of reads and the like.

Still another aspect of the present invention provides a method of selecting a candidate protein as a biomarker, the method comprising: (a) treating each of two samples to be analyzed including a test sample and a comparative sample with an aptamer library specific to a target protein population of any one of the two samples to be analyzed so as to form complexes between proteins of a target protein population of each sample and aptamers specifically binding thereto to thereby form a target protein-aptamer complex population in each sample, isolating the complex population from unbound aptamers in each sample, and converting aptamers of the isolated complex population in each sample into a double-stranded DNA population, (b) removing double-stranded DNA present in common between double-stranded DNA populations of the test sample and the comparative sample, from the double-stranded DNA population of the test sample, and (c) analyzing the remaining double-stranded DNA of the test sample, from which the double-stranded DNA present in common has been removed, through a next-generation sequencing process to thus analyze each double-stranded DNA sequence of the double-stranded DNA population and determine the abundance of each double-stranded DNA.

Also in the method of the present invention, step (c) may be performed by amplifying the remaining double-stranded DNA and analyzing the resultant amplification product using a next-generation sequencing process.

Also in the method of the present invention, step (b) may be performed through an SSH (suppression subtractive hybridization) process or a DSN (duplex-specific nuclease) process.

Yet another aspect of the present invention provides a method of simultaneously analyzing target nucleic acids and target proteins in a sample to be analyzed, suitable for simultaneously performing quantification of target proteins and sequencing and quantification of target nucleic acids, the method comprising: (a) (i) obtaining a protein sample containing a target protein population from a sample to be analyzed, treating the protein sample thus obtained with an aptamer library specific to the target protein population of the protein sample so as to form complexes between target proteins and aptamers binding specifically thereto to thereby form a target protein-aptamer complex population, isolating the complex population from unbound aptamers, and converting aptamers of the isolated complex population into double-stranded DNA, and (ii) obtaining a nucleic acid sample containing target nucleic acids from a sample the same as the sample to be analyzed and converting the target nucleic acids of the nucleic acid sample into double-stranded DNA fragments, (b) mixing the double-stranded DNA derived from the aptamers and the double-stranded DNA fragments derived from the target nucleic acids, and (c) analyzing each double-stranded DNA sequence in the mixture using a next-generation sequencing process, thus obtaining information on each double-stranded DNA sequence and determining the abundance of each double-stranded DNA.

In the method of the present invention, the target nucleic acids may be gDNA, RNA or a mixture thereof.

Also in the method of the present invention, the gDNA may be gDNA having at least one of sequence deletion, sequence insertion, single-nucleotide polymorphism (SNP), and cytosine methylation.

Also in the method of the present invention, the RNA may be mRNA, pre-mRNA, ncRNA (noncoding RNA) or a mixture thereof.

Also in the method of the present invention, the target proteins may be known proteins or unknown proteins, and the target nucleic acids may be known nucleic acids or unknown nucleic acids.

Also in the method of the present invention, sequencing libraries may be prepared from the double-stranded DNA of the aptamers or the double-stranded DNA fragments of the nucleic acids in step (a), and step (b) may be performed by mixing the sequencing libraries.

Also in the method of the present invention, the sample to be analyzed before treatment with the aptamer library in step (a) may be added with two or more external standard proteins having different quantification values that are absent in the sample, and the aptamer library in step (a) may use an aptamer library further including aptamers for the external standard proteins, whereby, in step (c), results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins.

Also in the method of the present invention, the sample to be analyzed before obtaining the nucleic acid sample in step (a) may be added with two or more external standard nucleic acids having different quantification values that are absent in the sample, and in step (c), quantification results for the external standard nucleic acids may be obtained, in addition to quantification results for the target nucleic acids, and the quantification results for the external standard nucleic acids and the quantification results for the target nucleic acids are compared, thereby quantifying the target nucleic acids.

Also in the method of the present invention, the target nucleic acids may be, in particular, pre mRNA or mRNA.

Also in the method of the present invention, the aptamer library may be obtained by (i) preparing an aptamer pool having a random sequence to thus have potential binding capacity to various proteins, (ii) reacting the aptamer pool with the target protein population of the same sample as in step (a) so as to induce specific binding between aptamers and target proteins to thereby form a complex population, (iii) isolating the complex population by excluding unbound aptamers, and (iv) amplifying the aptamers of the complex population.

Also in the method of the present invention, each aptamer of the aptamer library may have 5' and 3' regions comprising conserved regions of known sequences and a middle region therebetween comprising a variable region of any random sequence.

Also in the method of the present invention, the sample to be analyzed may be a processed sample obtained by removing a protein present in a large amount from the sample.

Also in the method of the present invention, the nucleic acid sample may be a nucleic acid sample having no rRNA.

Also in the method of the present invention, each aptamer of the aptamer library may have 5' and 3' regions comprising conserved regions of known sequences and a middle region therebetween comprising a variable region of any random sequence, whereby the double-stranded DNA population may be prepared using a set of a forward primer and a reverse primer.

Also in the method of the present invention, the aptamers may be single-stranded DNA or single-stranded RNA.

Also in the method of the present invention, the target proteins may be unknown proteins, known proteins, or a mixture of unknown proteins and known proteins. Preferably, when a certain protein of the target protein population is an unknown protein, isolating and identifying the unknown protein using an aptamer specific to the unknown protein that is contained in the aptamer library may be further performed.

Also in the method of the present invention, the quantifying the target proteins or the target nucleic acids in step (c) may be performed by counting the number of reads of the same sequence for double-stranded nucleic acids derived from the aptamers or double-stranded nucleic acid fragments derived from the target nucleic acids, counting the number of sequences that may be considered to be the same as the reads taking into account an error frequency of a next-generation sequencing process, and summing the number of reads and the number of sequences so that the target proteins or the target nucleic acids are quantified based on the summed values. Here, the number of reads and the like may be counted by comparing a reference sequence, which is a known sequence obtained by analyzing sequences of double-stranded nucleic acids derived from the aptamers or sequences of double-stranded nucleic acid fragments derived from the target nucleic acids, with sequence analysis results of double-stranded nucleic acids derived from the aptamers or sequence analysis results of double-stranded nucleic acid fragments derived from the target nucleic acids.

A detailed description of the present invention will be given below.

An aspect of the present invention pertains to a method of quantitatively analyzing a target protein population in a sample to be analyzed.

According to the present invention, the method of quantitatively analyzing the target protein population includes (a) treating a sample to be analyzed with an aptamer library specific to a target protein population present in the sample so as to form complexes between target proteins and aptamers binding specifically thereto, thereby forming a target protein-aptamer complex population, (b) isolating the complex population from unbound aptamers, and (c) analyzing the sequence of each aptamer of the complex population so as to quantify each aptamer of the complex population, thereby quantifying each target protein in the complex population.

In the method of the present invention, the aptamer library is reacted with the target protein population of the sample to be analyzed to thus form complexes between target proteins and aptamers specific thereto, thereby forming a complex population of a target protein population and an aptamer library population specific thereto, and each aptamer of the complex population is converted to double-stranded DNA and the double-stranded DNA for each aptamer is sequenced using a next-generation sequencing technique, ultimately quantitatively analyzing the target protein population.

Due to the Human Genome Project completed in the early 2000's, low-cost, high-speed, and large-capacity nucleic acid sequencing technology was required, and around the year 2007, products using NGS technology started to be marketed by Illumina (device name: Genome Analyzer HiSeq® series), Roche (device name: 454® series) and Life Technologies (device name: SOLiD® series). This NGS technology includes spatially separating a DNA library, obtained by fragmenting genomic DNA, into individual fragments on a substrate or emulsion (bead), amplifying the fragments using PCR to form clones of the fragments, and simultaneously sequencing hundreds of thousands to several billions of clones in a massively parallel manner to thus simultaneously read the sequences of the clones. This sequencing reaction is a method of physically and chemically detecting a signal resulting from attaching each mononucleotide through PCR (polymerase chain reaction) using a single DNA fragment of each clone as a template. Reads, which are sequence information obtained for the fragments, are compared with a reference sequence already analyzed, and aligned and combined through a bioinformatic technique to construct the entire genome sequence (NATuRe Genetics, 2010, 11:31-46; Trends in Genetics, 2008, 24(3):133-141; Genomics, 2008, 92:255-264). All documents cited herein, including these documents, are considered part of this specification.

When determining the sequences of aptamers that form the complexes using NGS in the method of the present invention, the number of reads of the same sequence or reads that may be considered to be the same sequence is counted, and the number of such reads reflects the abundance of the target proteins to which the aptamers specifically respond in the sample, ultimately realizing quantification of the target proteins.

However, NGS has a sequencing error frequency of about 0.1 to 2% due to the error caused by the polymerase during the PCR for the sequencing reaction and the error during the physicochemical detection of the signal, and this error frequency is known to be 1% (10⁻²) for Roche's 454 GS Junior, 0.1% (10⁻³) for HiSeq from Illumina and 2% (2×10⁻²) for SoLiD from Life Technologies (Fox et al., Next Generat. Sequenc. & Applic. 2014, 1:1).

Therefore, taking into account the error frequency of NGS, read sequences which are not the same sequence but may be considered to be the same sequence are deemed to be the same aptamer, and thus the accuracy of quantification of the target proteins to which the aptamers specifically bind may be increased.

In the method of the present invention, in order to more accurately quantify the target proteins to which the aptamers bind by regarding, as the same aptamer, the read sequences that may be considered to be the same sequence, the aptamer library uses a library of aptamers composed of 5' and 3' regions comprising conserved regions having known sequences and the middle region therebetween comprising a variable region having any random sequence different therefrom. Thereby, taking into account the error frequency of the NGS device, a sequence mismatch of 2% or less in the conserved region sequences is allowed, and thus the read sequences that are inconsistent as much as 2% or less with the conserved regions (including sequences that are exactly the same as the conserved regions) are determined as effective reads, and the remaining reads are excluded. The effective reads, having an inconsistent sequence of 2% or less in the variable region of the effective reads, are regarded as the same read (i.e. regarded as the same aptamer) and are thus used for the quantification of target proteins. When the error frequency of NGS is taken into account in this way, the accuracy of quantification of target proteins through sequencing of aptamers may be increased. The criterion for determining the effective reads taking into account such an error frequency may be appropriately adjusted in consideration of the error frequency of the NGS technique (or device) applied to the method of the present invention.

As used herein, the term "read" refers to the sequence of each double-stranded fragment analyzed by NGS. Each double-stranded fragment is separated from the aptamers. Since the aptamers have quantitative information on target proteins, the quantitative information on target proteins may be obtained by counting the number of reads of the same sequence or a sequence that may be considered to be the same sequence.

In the method of the present invention, the sample to be analyzed may be any sample in the form of a mixture or solution, which contains target proteins to be detected or is suspected of containing such target proteins to thus have the need for detection. The sample may be not only a biosample obtained from a human or an animal but also a processed sample in which the target protein concentration is increased by processing such a biosample, and may also be a sample that requires inspection, including environmental pollution factors, toxic factors, etc., such as water, food, industrial wastewater, etc., which contain or are suspected of containing target proteins. Such a sample may include an appropriate diluent or buffer solution.

In the method of the present invention, the sample to be analyzed is preferably a biosample obtained from a human or an animal or a processed sample thereof. A biosample may be obtained from a human or animal, which contains target proteins to be detected, such as blood, urine, saliva, semen, amniotic fluid, lymph fluid, sputum, tissue, synovial fluid, cells, cell extracts, etc., or is suspected of containing such target proteins to thus have the need for detection. Examples of the processed sample may include plasma, serum, a sample in which the protein concentration in a biosample is increased using a protein extraction kit, tissue extracts, cells obtained from tissue, cell lysate, cell broth, and the like. Furthermore, the processed sample may be a processed sample in which proteins that are present in large amounts in the biosample and are low in availability as target proteins (that is, having a low likelihood of being used as a biomarker for a certain disease) are removed from the biosample. For example, a very small number of some proteins in the blood sample accounts for 99.9% of proteins in the sample, and proteins present in such large amounts are typically low in availability as target proteins (Mol. Cell. Prot. (2006) 5(10):1727-1744). When a processed sample from which large amounts of proteins present in a biosample have been removed is used, the detection sensitivity of target proteins having high availability may be improved. Proteins present in such large amounts include, for example, albumin, IgG, IgA, transferrin, and fibrinogen in the case of blood samples of mammals including humans. Such large amounts of proteins may be removed using appropriate methods known in the art (such as immuno-affinity depletion) or using commercially available kits (e.g. Multiple Affinity Removal System from Agilent Technologies).

In the method of the present invention, the aptamer library in step (a) may be obtained, as shown in the following examples, by (i) manufacturing an aptamer pool having different sequences (i.e., random sequences) to thus have potential binding capacity to various proteins, (ii) reacting the aptamer pool with the target protein population of the same sample as in step (a) so as to induce specific binding between aptamers and target proteins to thereby form a complex population, (iii) isolating the complex population by excluding unbound aptamers, and (iv) amplifying the aptamers of the complex population.

In the process of preparing the aptamer library, the aptamer pool having a variety of different sequences in step (i) is generally a single-stranded RNA or DNA oligonucleotide pool, in which oligonucleotides generally comprise a 5' end conserved region and a 3' end conserved region comprising known sequences, and a variable region comprising a random sequence therebetween, as described above. The conserved regions of known sequences may include a sequence to which forward/reverse primers bind, a promoter sequence of RNA polymerase, a restriction enzyme recognition sequence for manipulation such as cloning, etc. Since the variable region comprising the random sequence is usually composed of 40 to 60 nucleotides, the entire oligonucleotide, including the 5' end region and the 3' end region, is typically 60 to 120 nucleotides in length. Synthesis of these oligonucleotides is well known in the art, and examples thereof include solid-phase oligonucleotide synthesis techniques, solution-phase synthesis techniques such as triester synthesis methods, and the like. Detailed content therefor is set forth in the paper [Nucl. Acid Res. 14:5399-5467, 1986], the paper [Tet. Lett. 27:5575-5578, 1986], the paper [Nucl. Acid Res. 4:2557, 1977], the paper [Lett., 28:2449, 1978], etc. Also, a commercially available automated DNA synthesizer may be used, and when such a synthesizer is used, an aptamer pool comprising a wide variety of aptamers, including 10¹⁴ to 10¹⁶ oligonucleotides, may be obtained. In order to use an RNA pool as the aptamer pool, the RNA pool obtained by transferring a DNA pool with RNA polymerase such as T3, T4, or T7 may be employed.

Also, in the preparation of the aptamer library, the unbound aptamers may be removed by any appropriate process known in the art for the isolation of the complex population of step (iii), for example, by performing a washing process one or more times using an appropriate washing buffer. After removal of the unbound aptamers and selective isolation of the complex population, the aptamers of the complex population may be amplified alone to thus manufacture an aptamer library. The aptamer library obtained by performing such selection and amplification only once may be used without change in step (a) of the method of the present invention, but the above selection and amplification may be repeated two or more times, and in particular, a series of processes, in which the aptamer library obtained by amplifying only the aptamers of the complex population is reacted again with the target protein population of the sample to be analyzed to form a complex population again, the complex population is isolated, and only the aptamers of the complex population are amplified again, is repeated two or more times, whereby an aptamer library having increased specific binding capacity to the target protein population of the sample to be analyzed may be used. However, it is desirable that the aptamer library be able to detect and analyze a greater diversity of target protein populations by variously reflecting the target proteins of the sample to be analyzed, and thus an aptamer library is preferably prepared through selection and amplification once, provided that a washing process is performed two or more times using various washing buffers, rather than repeating the above selection and amplification. The washing solution may be used by purchasing a product which is widely used in the art or through appropriate formulation, and such a washing solution generally includes a surfactant and/or a salt. Examples of the surfactant may include SDS, Tween 20, Tween 30, Tween 40, Tween 60, Tween 80, Triton X-405, Triton X-100, Tetronic 908, Cholesterol PEG 900, Polyoxyethylene Ether W-1, Span 20, Span 40, Span 85, and mixtures thereof, and examples of the salt may include lithium, sodium, potassium and ammonium acetates, lactates, citrates, phosphates, nitrates, sulfates, chlorides, and mixtures (SSC, SSPE, etc.) thereof. In particular, the washing solution may include a TBST solution (10 mM Tris-Cl, pH 8.0, 150 mM NaCl, 0.05% Tween 20), a PBST solution (PBS, pH 7.0, 0.05% Tween 20), or a SSPE solution (0.2 M phosphate buffer, 2.98 M NaCl, 20 mM EDTA, pH 7.4) including Tween 20 or Tween 30, and a 1-600 mM EDTA solution may also be used.

Also, in the method of the present invention, the term "target protein population" refers to a group of two or more different proteins. As described above, when the aptamer pool obtained by treating the sample to be analyzed with the aptamer pool to isolate the whole complex and amplifying the aptamers of the complex thus isolated is used as the aptamer library, a large number of target proteins may be detected collectively. Therefore, the target protein population may be understood as a protein population composed of at least 500 proteins, preferably 1000 or more proteins, more preferably 1500 or more proteins, and even more preferably 2000 or more proteins. Each aptamer of the aptamer pool may be an aptamer, the sequence of which is predetermined using the method of the present invention or using a sequencing process through cloning based on a known BAC library construction (Genome Res. 2001 Mar; 11 (3): 483-496), as necessary.

Also, in the method of the present invention, the term "aptamer library specific to a target protein population" in step (a) refers to a group of aptamers enabling detection of a target protein population by specifically binding to the target protein population. Accordingly, if the target protein population is composed of, for example, 1000 target proteins, the aptamer library will comprise at least 1000 aptamers. Here, "comprising at least 1000 aptamers" means that when an aptamer pool responds to any certain target protein, not only any one certain aptamer specifically binds to the target protein but also two or more certain aptamers may specifically bind to the target protein. In general, the aptamer library will contain more kinds of aptamers than the target proteins that make up the target protein population.

In the method of the present invention, the aptamer means a nucleic-acid ligand able to specifically bind to a target protein, like an antibody, and the aptamer may be a partial or complete double-stranded DNA or double-stranded RNA aptamer, so long as it may specifically bind to the target protein. Nevertheless, the aptamer is preferably a single-stranded DNA aptamer or a single-stranded RNA aptamer having high specific binding capacity, especially a single-stranded RNA aptamer. The single-stranded aptamer may be an aptamer chemically modified at the base position in order to resist chemical, physical, and enzymatic degradation. In the following examples of the present invention, an RNA pool was prepared, in which all C and U were fC (2'-F-modified C) and fU (2'-F-modified U) using 2'F-CTP and 2'F-UTP, and was used for the preparation of an aptamer library.

After step (a) of the method of the present invention, in which the target protein population of the sample to be analyzed and the aptamer library are reacted to form a complex population through specific binding between each target protein of the protein population and each aptamer of the aptamer library, the complex population needs to be isolated from the unbound aptamers in step (b) of the method of the present invention. The isolation of the complex population from the unbound aptamers may be conducted through any method known in the art. For example, the sample to be analyzed may be reacted with a reaction solution containing a nitrocellulose membrane having high binding affinity to proteins (a reaction solution may be composed of, for example, 60 mM Tris-Cl (pH 7.4), 5 mM KCl, 100 mM NaCl, 1 mM MgCl₂, and 0.1% NaN₃) to thus attach target proteins of the sample to the nitrocellulose membrane, followed by treatment with an aptamer library specific thereto to induce the formation of a complex and then performing washing using an appropriate washing buffer to thus remove unbound or nonspecifically bound aptamers, thereby recovering the nitrocellulose membrane to which only the complex is bound, ultimately enabling isolation of the complex population. Alternatively, for example, the isolation of such a complex population may be performed using a structure comprising a nitrocellulose filter and a nylon membrane. Particularly, a reaction solution containing an aptamer library is added with a sample and thus reacted, after which the reaction mixture solution is treated with a structure comprising a nitrocellulose filter and a nylon membrane and appropriate pressure is applied thereto, whereby the aptamer-protein complex remains on the nitrocellulose filter and the unbound single-stranded nucleic acids are present on the nylon membrane. The nitrocellulose filter is recovered and washed with an appropriate washing buffer to thus remove the unbound or nonspecifically bound aptamers, thereby attaining the complex population. Here, with regard to the washing buffer and the washing step using the same, reference may be made to the description of the preparation of the aptamer library above.

In the method of the present invention, after isolation of the complex population in step (b), step (c) is performed in a manner in which each aptamer sequence of the complex population is analyzed by the NGS technique and each aptamer of the complex population is quantified to thus quantify each target protein in the complex population. In order to analyze the sequence through the application of the NGS technique, it is necessary to convert the aptamers of the complex into double-stranded DNA fragments, which may be easily performed using techniques known in the art. For example, when the aptamers are single-stranded RNA aptamers, cDNA is produced through reverse transcription of the single-stranded RNA and is subjected to one-way PCR once. When it is required to appropriately increase the amount of the sample for sequence analysis using NGS technology or to detect target proteins present in trace amounts in the sample, cDNA may be subjected to PCR ones of times or tens of times to thereby increase the amount of the sample. When the sample is increased in its amount by repeating PCR ones of times or tens of times and is thus used as a sequencing library for NGS technology, the number of reads may increase in proportion to the number of times PCR is performed. The number of times PCR is performed may be taken into account when quantifying target proteins based on the number of reads.

PCR may be performed with a set of a forward primer and a reverse primer when the aptamer library of the present invention is a group of aptamers each having the 5' region and the 3' region composed of conserved regions having known sequences and the middle region therebetween composed of a variable region having any random sequence different therefrom.

In the method of the present invention, the sample to be analyzed before treatment with the aptamer library in step (a) is added with two or more external standard proteins having different quantification values (i.e. concentrations) that are absent in the sample, and the aptamer library in step (a) uses an aptamer library further including aptamers for the external standard proteins, whereby, in step (c), results of quantifying the aptamers for the external standard proteins are obtained, in addition to results of quantifying the aptamers for the target proteins, and the aptamer quantification results for the external standard proteins and the aptamer quantification results for the target proteins are compared, thereby making it possible to determine the quantification values of the target proteins. When the sample is added with the two or more external standard proteins having different quantification values (concentrations), a standard curve showing the correlation between the concentration of external standard proteins and the number of reads corresponding to the quantification values thereof is created, and the number of reads for the target proteins is substituted into the standard curve, thus enabling estimation of the concentration of the target proteins. Ones to hundreds of kinds of external standard proteins may be used in different quantification values. In this case, the quantification values of the target proteins may be estimated more accurately. It is preferred that not only the external standard proteins but also the analogues thereof be absent from the sample to be analyzed so that aptamers specifically binding thereto do not bind to the target proteins in the sample to be analyzed. The appropriate selection of such external standard proteins may be implemented by comparing the genetic information on biological species for which genome sequence analysis has been completed and the genetic information on biological species from which the sample to be analyzed is obtained. In the following examples of the present invention, the human-derived myocardial infarction patient serum was used as a sample to be analyzed, and the sample to be analyzed was added with five kinds of plant proteins derived from *Arabidopsis thaliana,* analogues of which are not likely to be present therein, at respective quantification values (concentrations) of 0.01 pg/mL, 1.0 pg/mL, 100.00 pg/mL, 10.0 ng/mL and 1.0 µg/mL. The use of the external standard proteins allows for quality control for variation in sample preparation (e.g. extraction of target proteins, etc.) and the like.

As used herein, the term "quantification" has a meaning including relative quantification and absolute quantification. "Relative quantification" means, for example, the ratio of each quantification value relative to the overall average of all quantification values or the ratio of each quantification value relative to a certain quantification value, and "absolute quantification" means the result of calculating the concentration using a standard curve between the quantification value and the concentration, like the case of using the above external standard proteins or the following external standard nucleic acids.

In the method of the present invention, the target protein population may be a population of unknown proteins, a population of known proteins, or a mixed population thereof.

In the method of the present invention, when a certain protein of the target protein population is an unknown protein, isolating the protein using an aptamer specific to the protein (which is contained in the aptamer library) and determining the amino acid sequence of the protein using a method known in the art (e.g. MALDI-TOF, etc.) to thus identify the protein may be further performed. Identification of these target proteins may provide useful disease-specific biomarker candidates.

In the method of the present invention, when the number of reads obtained from each aptamer is counted, as described above, reads of the same sequence and sequences that may be considered to be the same sequence taking into account NGS error frequency may be counted as reads of the same aptamer, but the number of reads may also be counted using, as a reference sequence, the sequence already determined for each aptamer of the aptamer library. Here, the reference sequence for each aptamer is preferably determined from the sequence already obtained for the same sample as the biosample to be analyzed through the method of the present invention described above.

Another aspect of the present invention pertains to a method of selecting a candidate protein as a biomarker by comparing the quantification results obtained from two samples to be analyzed using the above method. Here, the two samples to be analyzed are samples that are useful for comparison therebetween, for example, a sample of a patient (or a patient group) and a sample of a normal person (or a normal person group).

In the method of the present invention, the biomarker is primarily a biomarker that provides information on diseases afflicting mammals including humans. Such a biomarker differs in the presence and/or abundance thereof in samples between healthy individuals and diseased individuals, thereby enabling healthy individuals to be distinguished from diseased individuals. Also in the present invention, the biomarker is secondarily any biomarker that allows two samples to be distinguished from each other depending on the presence and/or abundance thereof in two samples to be analyzed (or biological species or biological individuals from which the samples are derived), thus providing information useful to humans, such as determining compliance with a drug prescription (e.g. anticancer companion diagnostics), medication adherence, the degree or severity of *in-vitro* cell responses to drug treatment, classification and identification of biological species, etc.

As used herein, the term "biomarker candidate" refers to a candidate that may be used for further study for discovery of a biomarker having a likelihood of being used as a biomarker. Such a biomarker candidate may be used as an actual biomarker if the availability thereof is confirmed in further studies. For example, a biomarker candidate for a certain disease may be used in clinical studies including the diseased patient group and the normal person group, and such a biomarker candidate may be used as a biomarker for diagnosis of such a disease when the availability thereof is confirmed by statistical significance through clinical studies.

The method of selecting the candidate protein as the biomarker of the present invention may include (a) treating a sample to be analyzed with an aptamer library specific to a target protein population present in the sample so as to form complexes between target proteins and aptamers binding specifically thereto, thereby forming a target protein-aptamer complex population, (b) isolating the complex population from unbound aptamers, and (c) analyzing the sequence of each aptamer of the complex population so as to quantify each aptamer of the complex population, thereby quantifying each target protein in the complex population, and may further include performing steps (a) to (c) for an additional sample to be analyzed different from the above sample to be analyzed and determining one or more target proteins having different quantification results by comparing the target protein quantification results obtained through step (c) between the two samples to be analyzed.

In the method of the present invention, the aptamer library in step (a) preferably uses the same aptamer library for the two samples to be analyzed. Here, the same aptamer library refers to an aptamer library having the same aptamer composition and concentration. When the same aptamer library is used, the aptamers for respective target proteins are identical (which are aptamers having the same sequence), and thus, since the binding capacities between the target proteins and the aptamers thereto are the same, there is no difference in the quantification results depending on the binding capacity, whereby the quantification results thus obtained may be used more effectively in the selection of the biomarker candidate.

Also, in the method of the present invention, like the method of quantitatively analyzing the target protein population as above, two or more external standard proteins having different quantification values (i.e. concentrations) present in neither of the two samples to be analyzed are added to each of the two samples to be analyzed, and quantification results for these external standard proteins may also be used to quantify the target proteins. More particularly, in this regard, reference may be made to the description of the method of quantitatively analyzing the target protein population according to the present invention.

Also, in the method of the present invention, when the target proteins are unknown proteins, isolating and identifying such proteins with aptamers specific thereto may be further performed. More particularly, in this regard, reference may be made to the description of the method of quantitatively analyzing the target protein population according to the present invention.

For technical details not described for the selection of the biomarker candidate protein of the present invention, the description of the method of quantitatively analyzing the target protein population according to the present invention may be applied without change, and reference may be made to the corresponding content.

Still another aspect of the present invention pertains to a method of more easily selecting a biomarker candidate protein by slightly changing the above method.

This method is performed in a manner in which double-stranded DNA, obtained through reverse transcription and PCR or through PCR from the aptamers for proteins present in common between the two samples to be analyzed, is removed from double-stranded DNA obtained from any one sample, and only double-stranded DNA is detected for the remaining proteins.

The proteins present in common between the two samples to be analyzed have low availability as biomarker candidate proteins. Briefly, the likelihood of use thereof as biomarkers is low. Therefore, the method of the present invention does not detect these proteins present in common.

Particularly, the method of the present invention includes (a) obtaining a protein sample containing a target protein population from each of two samples to be analyzed, treating the protein sample with an aptamer library specific to the target protein population of the protein sample so as to form complexes between target proteins and aptamers binding specifically thereto to thereby form a target protein-aptamer complex population, isolating the complex population from unbound aptamers, and converting aptamers of the isolated complex population into a double-stranded DNA population, (b) removing double-stranded DNA, present in common between the double-stranded DNA populations of the two samples to be analyzed, from a double-stranded DNA population obtained from any one of the two samples, and (c) analyzing the remaining double-stranded DNA population of the one sample using NGS technology to analyze each double-stranded DNA sequence and determine the abundance of each double-stranded DNA.

The double-stranded DNA present in common between the double-stranded DNA populations derived from the samples is double-stranded DNA for the protein present in common in the samples. When the double-stranded DNA present in common is removed, the remaining double-stranded DNA becomes double-stranded DNA specific to the sample (i.e. present only in the sample), thus reflecting information on proteins specific to the sample. These proteins may become biomarker candidate proteins. Here, it is preferred that the sample analyzed by removing the common double-stranded DNA be the relatively more useful sample among the two samples to be analyzed. For example, if the method of the present invention is intended to select a disease biomarker candidate protein, the sample is a sample derived from a patient (or patient group), and a sample derived from a normal person (or normal group) is the remaining sample. In the present specification including the claims, for the sake of convenience, a sample analyzed using NGS technology is referred to as a test sample and the remaining non-analyzed sample is referred to as a comparative sample.

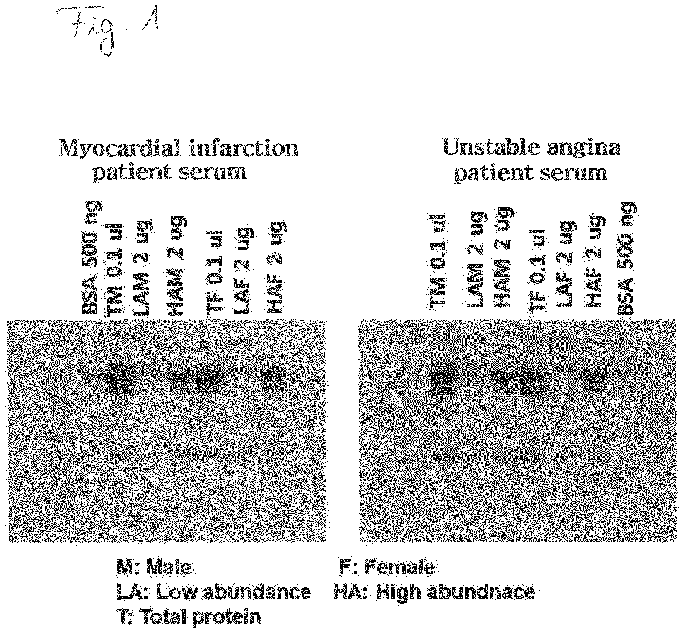

In the method of the present invention, removing the double-stranded DNA, present in common between the double-stranded DNA populations of the two samples to be analyzed, from the double-stranded DNA population obtained from the test sample may be performed using, for example, SSH (suppression subtractive hybridization). This SSH process was proposed in the paper [Proc. Natl. Acad. Sci. USA. 1996 Jun 11; 93(12): 6025-6030] by LUDA DIATCHENKO et al., and is characterized in that double-stranded DNA of the test sample is classified into Tester 1 and Tester 2, each of which is then added with an adapter specific thereto, the double-stranded DNA present in common between the test sample and the comparative sample is hybridized using the double-stranded DNA of the comparative sample as a driver, and only the DNA in the test sample is subjected to exponential amplification while preventing exponential amplification of the hybridized double-stranded DNA. Thereby, a product amplified with the double-stranded DNA specific to the test sample may be obtained. The paper of LUDA DIATCHENKO et al. is also considered part of this specification, like the other documents in this specification, and more detailed concepts and processes of the SSH method are described in FIG. 1 of the above paper or the accompanying drawings and examples of the present specification.

Also, in the method of the present invention, removing the double-stranded DNA present in common may be carried out using a DSN (duplex-specific nuclease) process. This process was proposed in the paper [Nucleic Acids Research, 2004, Vol. 32, No. 3 e37] by Bogdanova EA et al., in which the double-stranded DNA of a test sample and the double-stranded DNA of a comparative sample are hybridized and removed through DSN, and only the remaining double-stranded DNA is subjected to exponential amplification. Regarding the DSN process, reference may be made to the above paper and the accompanying drawings and examples of the present specification.

When the amplification product obtained by removing the double-stranded DNA present in common and amplifying only the remaining double-stranded DNA of the test sample is sequenced and quantified using NGS, the protein corresponding to the double-stranded DNA is present in a large amount only in the test sample in proportion to an increase in the quantification value of the double-stranded DNA, and thus a useful biomarker candidate may be obtained in descending order of quantification value.

For technical details not described for the other methods of the present invention, the description of the method of quantitatively analyzing the target protein population according to the present invention or the method of selecting the biomarker candidate protein according to the present invention may be applied without change, and reference may be made to the corresponding content.

Yet another aspect of the present invention pertains to a method of simultaneously analyzing target nucleic acids and target proteins using NGS.

The method of simultaneously analyzing target nucleic acids and target proteins in a sample to be analyzed using NGS according to the present invention includes (a) (i) obtaining a protein sample containing a target protein population from a sample to be analyzed, treating the protein sample thus obtained with an aptamer library specific to the target protein population of the protein sample so as to form complexes between target proteins and aptamers binding specifically thereto to thereby form a target protein-aptamer complex population, isolating the complex population from unbound aptamers, and converting aptamers of the isolated complex population into double-stranded DNA, and (ii) obtaining a nucleic acid sample containing target nucleic acids from a sample the same as the above sample and converting the target nucleic acids of the nucleic acid sample into double-stranded DNA fragments, (b) mixing the double-stranded DNA derived from the aptamers and the double-stranded DNA fragments derived from the target nucleic acids, and (c) analyzing each double-stranded DNA sequence in the resultant mixture using NGS, thus obtaining information on each double-stranded DNA sequence and determining the abundance of each double-stranded DNA.

In the simultaneous analysis method of the present invention, the target proteins and the target nucleic acids in a sample may be simultaneously analyzed using NGS technology, which enables the sequence analysis of nucleic acids and the quantification of nucleic acids having the same sequence. To this end, the aptamers for the target proteins in the sample are converted into double-stranded DNA to which the NGS process may be applied, and also, the nucleic acids in the sample are converted into double-stranded nucleic acid fragments, followed by mixing and NGS, thereby simultaneously analyzing the proteins and the nucleic acids in the sample. When simultaneous analysis is performed using NGS, quantitative information on the target proteins and the target nucleic acids of a certain sample to be analyzed may be obtained simultaneously, and moreover, the sequences of the aptamers specific to the target proteins and the sequences of the target nucleic acids may be obtained.

In the simultaneous analysis method of the present invention, the sample to be analyzed may be any mixture or solution, which contains target proteins and target nucleic acids to be detected or is suspected of containing such target proteins and target nucleic acids to thus have the need for detection. It is preferably a biosample. For technical details for the other samples to be analyzed, the description of the method of quantitatively analyzing the target protein population according to the present invention may be applied without change, and reference may be made to the related content.

Also in the simultaneous analysis method of the present invention, for the isolation of the complex population from the unbound aptamers in step (a)(i), the description of the method of quantitatively analyzing the target protein population according to the present invention may be applied without change, and reference may be made to the related content.

Also in the simultaneous analysis method of the present invention, converting the aptamers of the complex population into the double-stranded DNA in step (a)(i) may be performed as follows: for the aptamers of the complex population, the aptamers are subjected to reverse transcription when being single-stranded RNA aptamers to thus obtain single-stranded cDNA, which is then subjected to one-way PCR once. When the aptamers are single-stranded DNA, one-way PCR may be directly performed once. Moreover, when the amount of the sample needs to be appropriately increased in order to perform sequence analysis using NGS, the amount of the sample may be increased by repeating PCR ones of times or tens of times on cDNA or the like. Such reverse transcription and/or PCR may be carried out on the aptamer population that is dissociated from the complex population, but reverse transcription and PCR include a heating process, and thus the aptamer population is dissociated from the complex population, and the above additional dissociation process may therefore be obviated. Here, PCR may be performed using a set of primers when forward and reverse primer binding sites are composed of conserved regions of known sequences, as described with regard to the method of quantitatively analyzing the target protein population according to the present invention. The double-stranded DNA derived from the aptamers is a fragment having a size that is already suitable for the application of NGS, and thus fragmentation thereof is not particularly required.

In the simultaneous analysis method of the present invention, the target nucleic acids include gDNA and/or RNA. gDNA includes gDNA having epigenetic changes such as deletion, insertion, single-nucleotide polymorphism (SNP), methylation, etc. When methylated gDNA is to be analyzed, it may be appropriately pretreated through methods known in the art (such as bisulfite treatment) and used for NGS analysis. For DNA methylation analysis using NGS, reference may be made to the paper [Cancer Res. 67 (2007) 8511-8518], the paper [Cancer Metastasis Rev (2011) 30:199-210], the paper [Biology (Basel). 2016 Mar; 5(1):3], the paper [J Vis Exp. 2015; (96): 52488] and the like, and for NGS analysis through insertion, deletion, SNP and the like, reference may be made to the paper [Cancer Genet. 2013 Dec; 206(12): 432-440], the paper [Front Bioeng. Biotechnol. 2015; 3: 92], the paper [PLoS One, 2014 9: e104652], the paper [Nature Reviews Genetics, 2011, 12:443-451] and the like. gDNA is separated using methods known in the art (Molecular Ecology Notes (2003) 3, 317-320; J Forensic Sci., May 2009, 54(3):599-607), such as a phenol-chloroform extraction method, etc. or commercially available kits (e.g. DNAzol™ Reagent, PureLink™ Genomic DNA Mini Kit, etc.), and is then subjected to sonication and thus randomly fragmented to a predetermined length (typically 1 kb or less), thereby analyzing the sequence thereof using NGS.

When the target nucleic acids are RNA, examples thereof include mRNA encoding protein, pre-mRNA before splicing, snRNA (small nuclear RNA) involved in splicing, snoRNA (small nucleolar RNA) involved in the modification of rRNA, miRNA (micro RNA) or piRNA (piwi-interacting) involved in the regulation of gene expression in the post-transcriptional stage, etc. Using methods known in the art (J Mol Diagn. 2008 May; 10(3): 203-211; PREPARATIVE BIOCHEMISTRY & BIOTECHNOLOGY Vol. 34, No. 3, pp. 209-214, 2004) or commercially available kits (TRIZOL™ Reagent, RNeasy™ FFPE Kit, PureLink™ FFPE RNA Isolation Kit, RecoverAll™ Total Nucleic Acid Isolation Kit), total RNA or RNA to be analyzed (e.g. mRNA) is separated and converted into double-stranded cDNA, the sequence of which is then analyzed and quantified using NGS. Preferably, when total RNA of the biosample is separated and is to be used as a sample to which NGS may be applied, rRNA, which occupies most of the total RNA (usually 95%), is removed using methods known in the art or commercially available kits (RiboMinus™, RiboZero™), whereby the remaining relatively small amount of RNA in total RNA, for example, mRNA, pre-mRNA, ncRNA (noncoding RNA) such as snRNA or miRNA, etc. may be more easily subjected to sequencing and quantification using NGS. When only mRNA is to be analyzed as the target nucleic acid, mRNA contains polyA and thus NGS analysis may be performed by isolating mRNA using polyT oligos immobilized on a support such as magnetic beads, and when only small RNA such as miRNA is to be analyzed as the target nucleic acid, miRNA is isolated through size selection using gel electrophoresis and may then be analyzed using NGS. For long RNA, such as mRNA and pre-mRNA, it is preferably fragmented to an appropriate size for the application of NGS technology before or after cDNA synthesis, and for a relatively small amount of RNA, an amplification product obtained by amplifying cDNA thereof is preferably analyzed using NGS.

Also, the target nucleic acid may be any gene in gDNA having an unknown sequence, or may be any RNA such as mRNA having an unknown sequence, or alternatively, may be a certain gene or RNA having a known sequence or an amplification product obtained from such a gene or RNA. In this way, the target nucleic acid may be any nucleic acid in the sample that requires sequencing and/or quantification.

Also, in the simultaneous analysis method of the present invention, for the double-stranded DNA of the aptamers or the double-stranded DNA fragments of the nucleic acids in step (a), each sequencing library is prepared through adapter addition, etc., and then mixed, and the sequencing library mixture may be analyzed using an NGS device. In this regard, when describing an NGS device from Illumina as an example, DNA fragments of the sequencing library are prepared by end repair, adenosine conjugation (dA-Tailing), and adapter addition. The sequencing library is intended to convert the DNA fragment library into a state capable of performing sequence analysis using an NGS device, and although the NGS device of Illumina was used as an example in the above description, double-stranded DNA fragments may be converted into a sequencing library in accordance with the manufacturer's protocol for each NGS device.

Also, in the simultaneous analysis method of the present invention, before mixing the samples in step (a), for example, the double-stranded DNA of the aptamers and the double-stranded DNA fragments of the nucleic acids, a barcode capable of distinguishing the samples from which they are derived may be added to the double-stranded DNA with the addition of an adapter. Upon simultaneous analysis of gDNA and RNA with the target proteins, such a barcode may be introduced to the double-stranded DNA fragments obtained from each sample in order to distinguish these nucleic acid samples, and upon RNA analysis, such a barcode may be introduced to the double-stranded DNA fragments obtained from each sample in order to distinguish RNA samples (e.g. a small RNA sample such as miRNA and an mRNA sample) . These barcodes, which are DNA of short length (usually 6 bp), are made to have a unique sequence for each sample so that samples may be distinguished. The distinction of samples using such barcodes in sequence analysis of multiple samples via NGS is well known in the art, and reference may be made to the paper [Mol. Ecol. 19, 2455-473 (2010)], the paper [Biology 2012, 1(3), 895-905], etc.

Also, in the simultaneous analysis method of the present invention, like the method of quantitatively analyzing the target protein population according to the present invention, the sample to be analyzed before classification into the protein sample and the nucleic acid sample in step (a) is added with two or more external standard proteins having no analogues at different quantification values, and aptamers specific to the external standard proteins are contained in the aptamer library, whereby the detection results for the external standard proteins may be used to quantify the target proteins. For a detailed description thereof, reference may be made to the description of the method of quantitatively analyzing the target protein population according to the present invention.

Also, in the simultaneous analysis method of the present invention, as with the use of two or more external standard proteins for the quantification of target proteins, in order to quantify nucleic acids, the sample to be analyzed in step (a) may be added with two or more external standard nucleic acids, which are not present in the sample, at different quantification values. In this case, the quantification values of the external standard nucleic acids may be useful for quantification of the target nucleic acids. Such external standard nucleic acids may be prepared in an appropriate size corresponding to the target nucleic acids in consideration of the target nucleic acids to be quantified. For example, when the target nucleic acids are mRNA and have a size of 1 kb, the external standard nucleic acids may be prepared at 800b to 1200b. Furthermore, the external standard nucleic acids may be the same kinds of nucleic acids; namely, when the target nucleic acids are mRNA, the external standard nucleic acids may be made into RNA in which polyA is conjugated to the 3' end. Appropriate selection of the external standard nucleic acids may also be achieved by comparing the genetic information on biological species for which genome sequence analysis has been completed and the genetic information on biological species from which the sample to be analyzed is obtained, as in the external standard proteins as described above. These external standard nucleic acids may also be used for quality control for variation in sample preparation, etc., like the external standard proteins. So long as two or more external standard nucleic acids are used, several kinds to hundreds of kinds may be used at different quantification values without any particular limitation.

In the simultaneous analysis method of the present invention, like the method of quantitatively analyzing the target protein population according to the present invention, the reference sequence already determined corresponding to each target nucleic acid as well as the aptamer of each target protein may be used for the quantification and the sequencing in step (c). When the target protein is an unknown protein, isolation and identification may be further performed using an aptamer thereto.

For technical details not described for the simultaneous analysis method of the present invention, the description of the method of quantitatively analyzing the target protein population according to the present invention may be applied without change, and reference may be made to the corresponding content.

### Advantageous Effects

As described hereinbefore, a method of quantitatively analyzing a target protein population in a sample to be analyzed using NGS according to the present invention, etc. can be provided.

### Description of Drawings

FIG. 1 shows the results of electrophoresis after removal of proteins present in large amounts from human serum protein samples;
FIGS. 2 and 3 are a schematic view and a flowchart showing a process of preparing a secondary library by performing SSH on a molecule-binding nucleic acid primary library;
FIG. 4 is a flowchart of a process of preparing a secondary library from a molecule-binding nucleic acid primary library using DSN;
FIG. 5 shows the electrophoresis results of the extent of subtraction of the secondary library obtained using DSN;
FIG. 6 shows the results of calculation of the appearance frequencies and the base sequences of the molecule-binding nucleic acids constituting the molecule-binding nucleic acid primary library in an analytical sample S and a comparative sample C;
FIG. 7 is a graph showing the distribution of 100 molecule-binding nucleic acids obtained by analyzing the myocardial infarction patient serum as an analytical sample and the unstable angina patient serum as a comparative sample with 1,149 molecule-binding nucleic acids of the molecule-binding nucleic acid secondary library;
FIG. 8 shows the results of clustering of samples using 100 molecule-binding nucleic acids obtained by analyzing the myocardial infarction patient serum as an analytical sample and the unstable angina patient serum as a comparative sample with 1,149 molecule-binding nucleic acids of the molecule-binding nucleic acid secondary library;
FIG. 9 shows the electrophoresis results (a) of certain molecule-binding nucleic acids contributing to the biological meaning analysis in the myocardial infarction patient serum as an analytical sample and the unstable angina patient serum as a comparative sample and the graph (b) of the electrophoretic band densities thereof;
FIG. 10 shows the results of observation of anticancer-drug-treated cells and untreated cells by labeling, with a fluorescent substance, molecule-binding nucleic acids selected by analyzing the extent of binding of anticancer-drug-treated cells and untreated cells to proteins;
FIG. 11 shows the results of evaluation of the selected liver-cancer-cell-line-specific molecule-binding nucleic acids specifically binding to a liver cancer cell line (Hep3B); and
FIG. 12 shows the analysis results of cdk2 mRNA after treatment of a liver cancer cell line with a Hep3B molecule-binding nucleic acid-siRNA cdk2 complex through various processes.

### Mode for Invention

A better understanding of the present invention will be given through the following examples. These examples are merely set forth to illustrate the present invention but are not to be construed as limiting the scope of the present invention.

### <Example 1> Preparation of single-stranded nucleic acid library

### <Example 1-1> Oligonucleotide of single-stranded nucleic acid library and primer

In order to prepare a single-stranded nucleic acid library reacting with a biosample (an analytical sample and a comparative example) containing a target protein population, oligonucleotides of <General Formula I> below (random single-stranded nucleic acids) were prepared (Bionia, Korea).

The oligonucleotides constituting the single-stranded nucleic acid library, as represented in <General Formula I>, were composed of 5' conserved region - variable region - 3' conserved region.

### <General Formula I> Oligonucleotide structure:

5'-
GGGAGAGCGGAAGCGTGCTGGGCCN50CATAACCCAGAGGTCGATGGATCCCCCC-3'

The base sequences underlined above are conserved regions, which are fixed portions comprising the known sequences of the single-stranded nucleic acid library, and 50 bases N₅₀, corresponding to the variable region, include adenine (A), guanine (G), thymine (T), and cytosine (C) present at the same concentration at individual positions.

A double-stranded DNA library was prepared by performing PCR using the oligonucleotide of <General Formula I> as a template. Here, the primers used are a DS forward primer (SEQ ID NO: 1) and a DS reverse primer (SEQ ID NO: 2) as represented below.
DS forward primer: 
5′-GGGGCTAATACGACTCACTATAGGGAGAGCGGAAGCGTGCTGGG-
3′ (SEQ ID NO: 1)
DS reverse primer:
   5'-GGGGCATCGACCTCTGGGTTATG-3' (SEQ ID NO: 2)

The DS forward primer (SEQ ID NO: 1) may complementarily bind to the 5'-end underlined sequence of the single-stranded DNA oligonucleotide of <General Formula I>. Furthermore, the underlined portion of SEQ ID NO: 1 is a T7 promoter sequence for RNA polymerase of bacteriophage T7.

The DS reverse primer (SEQ ID NO: 2) used for PCR may complementarily bind to the 3'-end underlined sequence of the single-stranded DNA oligonucleotide of <General Formula I>.

PCR (polymerase chain reaction) was performed using the DS forward primer (SEQ ID NO: 1) and the DS reverse primer (SEQ ID NO: 2) and using the single-stranded nucleic acid library of <General Formula I> as a template.

Particularly, 1,000 pmol single-stranded nucleic acid library and 2,600 pmol DS primer pair (a DS forward primer, a DS reverse primer) were mixed with 60 mM KCl, 10 mM Tris-Cl (pH 8.3), 3 mM MgCl₂, 0.5 mM dNTP (dATP, dCTP, dGTP, and dTTP) and 0.1 U Taq DNA polymerase (Perkin-Elmer, Foster City Calif.), and PCR was performed, followed by purification with a QIAquick-spin PCR purification column (QIAGEN Inc., Chatsworth Calif.). Thereby, double-stranded DNA containing the T7 promoter was prepared.

The corresponding PCR product is double-stranded DNA containing the T7 promoter, and the general formula thereof is represented by <General Formula II> below.

### <General Formula II> PCR product:

5'-
GGGGGCTAATACGACTCACTATAGGGAGAGCGGAAGCGTGCTGGGCCN50CATAACCCAGA
GGTCGATCCCC-3'

As will be described below, the primers for RT-PCR are an "RS forward primer (SEQ ID NO: 3)" and an "RS reverse primer (SEQ ID NO: 4)", and the base sequences thereof are as follows.
RS forward primer:
   5'-CGGAAGCGTGCTGGGCC-3' (SEQ ID NO: 3)
RS reverse primer:
   5'-TCGACCTCTGGGTTATG-3' (SEQ ID NO: 4)

### <Example 1-2> Preparation of single-stranded RNA library

A single-stranded RNA library reacting with a biosample (an analytical sample and a comparative sample) was prepared. This single-stranded RNA library is an RNA library containing 2'-F-substituted pyrimidine as the modified nucleotide, and single-stranded RNA containing 2'-F-substituted pyrimidine was prepared through purification after synthesis through *in-vitro* transcription using the PCR product of <General Formula II> prepared in <Example 1-1> by means of a DuraScribe T7 Transcription Kit (EPICENTRE, USA).

Particularly, 300 pmol double-stranded DNA prepared as above, 50 mM Tris-Cl (pH 8.0), 12 mM MgCl₂, 5 mM DTT, 1 mM spermidine, 0.002% Triton X-100, 4% PEG 8000, 5U T7 RNA polymerase, 1 mM (ATP, GTP) and 3 mM (2'F-CTP, 2'F-UTP) were mixed, reacted at 37°C for 6 to 12 hr, and purified with a Bio-Spin 6 chromatography column (Bio-Rad Laboratories, Hercules Calif.), after which the amount of the purified nucleic acids and the purity thereof were analyzed using a UV spectrometer.

### <Example 1-3> Preparation of external standard material for quality control and measurement

As external standard proteins, five kinds of plant (*Arabidopsis*)-specific proteins, namely A, B, C, D and E, analogues of which are not present in humans, secured from Plant Genomics of Michigan State University (http://genomics.msu.edu/plant specific/), were prepared using an *Escherichia coli* expression system, as shown in [Table 1] below.

**[Table 1]**

| Plant-specific protein | | | |
|---|---|---|---|
| Kind | Locus | Description | Accession number |
| A | At1g65390.1 | defense/immunity protein | GO:0003793 |
| B | At5g39310.1 | cell elongation | GO:0009826 |
| C | At4g15910.1 | Drought-Induced Protein (Di21) | GO:0009414 |
| D | At1g12860.1 | Bhlh Protein | GO:0003677 |
| E | At4g02540.1 | Chloroplast Thylakoid Lumen Protein | GO:0009543 |

### <Example 2> Preparation of molecule-binding nucleic acid primary library

### <Example 2-1> Preparation of molecule-binding nucleic acid primary library

In order to prepare a molecule-binding nucleic acid primary library, a biosample containing a protein population, for example, the serum, was used as a sample, and the myocardial infarction patient serum was used as an analytical sample and the unstable angina patient serum was used as a comparative sample.

In order to increase the detection sensitivity of useful target proteins, a sample obtained by removing excess protein present in the patient serum using a MARC column (Agilent Technologies Inc. USA) in accordance with the protocol provided by the manufacturer was used for actual experiments. The electrophoresis results of the excess-protein-free analytical sample and comparative sample are shown in FIG. 1.

In order to prepare a molecule-binding nucleic acid primary library, a single-stranded RNA pool specific to the protein population of the analytical sample (obtained by removing excess protein present in the myocardial infarction patient serum) was made, and the specific single-stranded RNA pool was allowed to react with the excess-protein-free analytical sample and comparative sample, thus preparing a molecule-binding nucleic acid primary library for each sample, which was then used to construct a sequencing library for NGS.

For the preparation of the single-stranded nucleic acid pool specific to the sample, the single-stranded RNA library synthesized in <Example 1> was allowed to react with the analytical sample obtained by removing excess protein present in the myocardial infarction patient serum to give a protein-single-stranded RNA complex, followed by repeating a washing process using the same washing buffer, thereby removing unbound or nonspecific single-stranded RNA. Next, the complex pool was isolated, and a single-stranded RNA pool obtained by dissociating the single-stranded RNA from the complex was amplified through RT-PCR (reverse transcription-PCR) using the RS forward primer and the RS reverse primer of <Example 1>, after which the resulting amplification product was subjected to *in-vitro* transcription in the same manner as in Example 1, thereby obtaining a single-stranded RNA pool.

The process of preparing the single-stranded RNA pool is described in detail below.

A reaction solution (50 mM HEPES, pH 7.5, 125 mM NaCl, 5 mM KCl, 5 mM NgCl₂, 1 mM EDTA, and 0.05% TWEEN-30) containing 300 µL of a 10¹⁴ base sequence/mL solution of the 300 pmol RNA library prepared in <Example 1-2> was heated at 80°C for 10 min and then allowed to stand in ice for 10 min.

This reaction solution was added with yeast tRNA (Life Technologies) in an amount five times the amount of the single-stranded nucleic acids used above and 0.2% BSA (bovine serum albumin, Merck), thus affording a nonspecific-reaction-blocking buffer solution.

Before immobilization of the serum protein on an NC (nitrocellulose) membrane piece (0.3 × 0.3 mm²), the reaction solution containing the RNA library was added with 60 µL of 100 mM DTT and the nonspecific-reaction-blocking buffer solution was added with 400 µL of 100 mM DTT and 10% BSA.

60 µL of the reaction solution (50 mM HEPES, pH 7.5, 125 mM NaCl, 5 mM KCl, 5 mM NgCl₂, 1 mM EDTA, 0.05% TWEEN-30) and 600 µg of the prepared serum protein were mixed in a 1.5 mL binding reaction tube. Tapping and then quick-spinning were performed so that the NC membrane was completely immersed in the reaction solution. Mixing was performed using a stirrer for 40 min at 100 rpm.

The NC membrane was dried at room temperature for 10 min and then placed in a new 1.5 mL tube. 300 µL of the nonspecific-reaction-blocking buffer solution was added thereto and mixed using a stirrer at 100 rpm for 40 min and the NC membrane was then transferred into a new 1.5 mL tube. 500 µL of the reaction solution was added thereto and mixed using a stirrer at 100 rpm for 10 min and the NC membrane was then transferred into a new 1.5 mL tube. Further, 500 µL of the reaction solution was added thereto and mixed using a stirrer at 100 rpm for 10 min and the NC membrane was then transferred into a new 1.5 mL tube. Thereafter, a washing process for removing the unbound serum protein was performed using the reaction solution.

5 µL of the reaction solution (100 ng/µL) containing the single-stranded RNA pool and 195 µL of a binding buffer solution were added thereto and mixed using a stirrer at 300 rpm for 40 min and the NC membrane was then transferred into a new 1.5 mL tube. Further, 500 µL of the reaction solution was added thereto and mixed using a stirrer at 100 rpm for 10 min and the NC membrane was then transferred into a new 1.5 mL tube. Furthermore, 500 µL of the reaction solution was added thereto and mixed using a stirrer at 100 rpm for 10 min and the NC membrane was then transferred into a new 1.5 mL tube.

The NC membrane was placed on a Whatman filter paper and dried at room temperature for 10 min. The dried NC membrane was placed in a new 1.5 mL tube, added with 50 µL of DEPC sterile purified water, and allowed to stand in a heat block at 95°C for 10 min, and thus RNA was eluted. RNA attached to the membrane was eluted through tapping, subjected to quick-spinning and then allowed to stand in ice. The RNA thus obtained was placed in a PCR tube and subjected to reverse transcription and PCR at 36°C using the RS forward primer and the RS forward primer of <Example 1> and then to *in-vitro* transcription in the same manner as in <Example 1>, thereby preparing a single-stranded RNA pool. The molecule-binding nucleic acid primary library binding to the human serum sample protein was prepared in a manner in which procedures of reacting the proteins of a human serum sample and the RNA library and performing washing using various washing buffers (0 to 1× reaction solution, 0 to 5% Tween-30 or 0 to 600 mM EDTA solution) were repeated once.

A molecule-binding primary single-stranded nucleic acid library was prepared in a manner in which the single-stranded RNA pool obtained above was allowed to react with each of the analytical sample and the comparative sample to thus remove unbound or nonspecific single-stranded RNA, thereby separating the protein-complex pool, after which the single-stranded RNA was dissociated from the complex pool. Here, the washing process was performed only once.

Next, the molecule-binding single-stranded RNA was subjected to reverse transcription to afford cDNA, which was then subjected to one-way PCR once, thus obtaining double-stranded DNA fragments, which were then subjected to NGS analysis as below.

### <Example 2-2> Preparation of molecule-binding nucleic acid primary library for sample containing external standard protein

In order to use the external standard proteins of Example 1 to quantify the target proteins of the analytical sample or the comparative sample, single-stranded RNA specifically binding to each external standard protein was attained using the single-stranded RNA library synthesized in <Example 1> through a standard SELEX method (Ellington, A.D. and J.W. Szostak. 1990. In vitro selection of RNA molecules that bind specific ligands. Nature 346: 818-822; Gold, L., P.Allen, J. Binkley, D. Brown, D. Schneider, S.R. Eddy, C. Tuerk, L. Green, S. Macdougal, and D. Tasset. 1993. RNA: the shape of things to come, pp. 497-510. In: R.F. Gestelend and J.F. Atkins (eds.). The RNA World, Cold Spring Harbor Press, Cold Spring Harbor, N.Y.), followed by a sequencing process through cloning based on a known BAC library construction (Genome Res. 2001 Mar; 11(3):483-496), thereby predetermining the sequences thereof.

Five kinds of external standard proteins prepared in <Example 1>, A, B, C, D and E were added at concentrations of 0.01 pg/mL, 1.0 pg/mL, 100.0 pg/mL, 10.0 ng/mL, and 1.0 µg/mL, respectively, to each of the analytical sample and the comparative sample to afford samples to be analyzed for the present example. Also, the single-stranded RNA pool reacting with these samples was added with single-stranded RNA specifically binding to each external standard protein, thus preparing a single-stranded RNA pool reacting with the sample. Each of the samples added with the five kinds of external standard proteins at different concentrations was reacted with the single-stranded RNA pool containing the single-stranded RNA specific to the external standard proteins to afford a molecule-binding nucleic acid primary library in the same manner as in <Example 2-1>, which was then subjected to reverse, transcription and one-way PCR once, and the resulting product was subjected to NGS analysis, as will be described below.

### <Example 3> Preparation of molecule-binding nucleic acid secondary library

### <Example 3-1> Preparation of molecule-binding nucleic acid secondary library using SSH

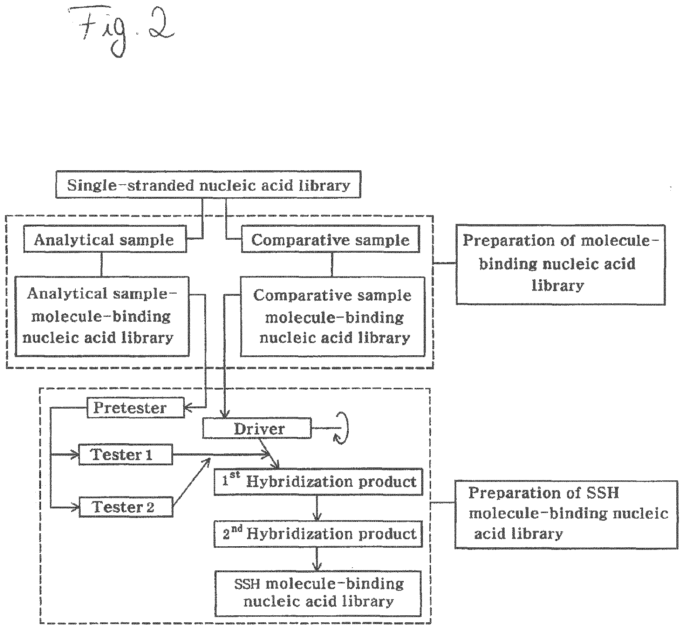

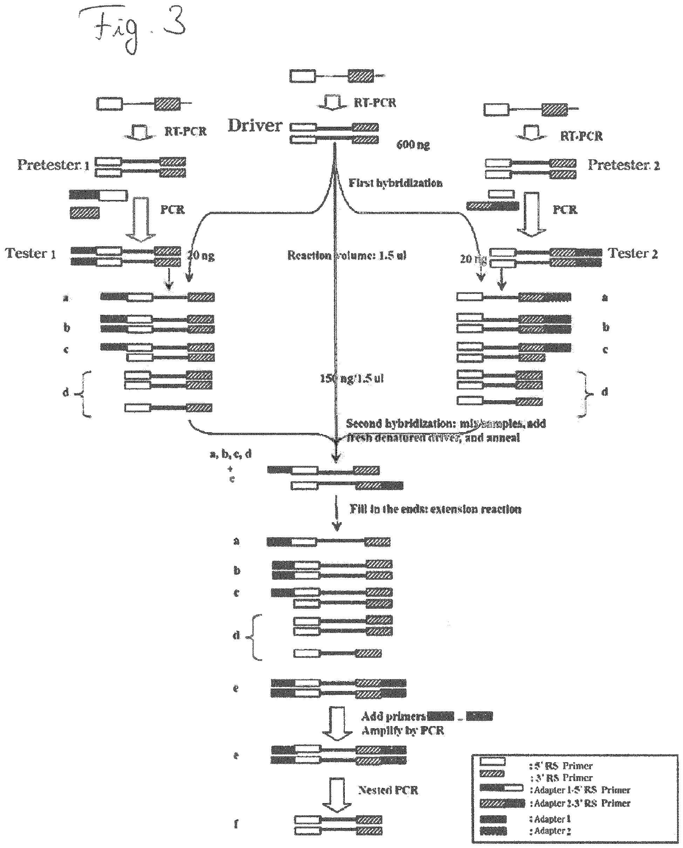

The preparation of the SSH molecule-binding nucleic acid secondary library was performed in the steps shown in [FIG. 2] and [FIG. 3].

RT-PCR was performed using, as a template, the molecule-binding primary RNA library for the proteins of the analytical sample prepared in <Example 2> and the molecule-binding primary RNA library for the proteins of the comparative sample, thereby preparing a DNA pool. Here, RT-PCR was conducted through a standard method.

Particularly, in order to carry out reverse transcription, 36 µL of a reaction solution containing the primary library was placed in a PCR tube, and 10 µL of a 5× reverse transcription buffer solution and 1 µL of an RS reverse primer (25 pmol/µL) solution were added thereto and mixed together. The resulting mixed solution was reacted at 65°C for 5 min and then at 25°C for 10 min using a PCR device. A mixed solution comprising 1.5 µL of DEPC sterile purified water, 1 µL of 30 mM dNTP, and 0.5 µL of AMV RTase (10 µ/µL BEAMS, Cat. No.4001L) was prepared, and 3 µL thereof was then added to the PCR tube in which the reaction was completed, so that the final volume was 60 µL. The reaction in the PCR tube was carried out using a PCR device at 37°C for 40 min and at 95°C for 5 min.

Subsequently, PCR was performed as follows. In order to amplify the product cDNA, PCR amplification was carried out using a PCR mixed solution prepared in a manner in which 60 µL of the RT reaction product was added with 10 µL of a 10× PCR buffer solution, 1 µL of 30 mM dNTP, 1 µL of a DS forward primer (25 pmol/µL), 1 µL of a DS reverse primer (25 pmol/µL), 0.5 µL of Taq polymerase (5 µ/µL) and 36.5 µL of tertiary sterile purified water so that the final volume was 100 µL. A series of procedures of preliminary denaturation at 95°C for 5 min, denaturation at 95°C for 40 sec, binding at 55°C for 40 sec, and extension at 72°C for 40 sec was repeated 25 times using a PCR device, and final extension at 72°C for 5 min was performed.

The PCR product was transferred into a 1.5 mL tube, 100 µL of deionized water was added so that the volume was increased to 300 µL, and phenol, chloroform and isoamyl alcohol were added in 300 µL of the same volume. After vortex mixing and then centrifugation at 13,000 rpm for 1 min, the supernatant was transferred into a new 1.5 mL tube. A two-fold volume of 100% ethanol and 0.1-fold volume of 3 M sodium acetate (pH 5.2) were added thereto and mixed, and the tube was stored at -30°C for at least 60 min or at 80°C for at least 10 min. The tube thus stored was centrifuged at 4°C and at 13,000 rpm for 10 min, and the supernatant was removed. 600 µL of 70% ethanol was added thereto, followed by centrifugation at 4°C and at 13,000 rpm for 5 min, after which the supernatant was removed.

The remaining content of the tube was dried and dissolved in 40 µL of tertiary sterile purified water. Then, 5 µL thereof was loaded on a 2.5% agarose gel and 3 µL of a 60 bp ladder DNA marker was then loaded thereon, followed by quantification in a 100 bp band using an Alphaimager program (BIO-RAD, USA).

The PCR product of the molecule-binding nucleic acid primary library binding to the proteins of the analytical sample was used as a "pretester", and a tester for use in SSH was prepared from the pretester, and the PCR product of the molecule-binding nucleic acid primary library binding to the proteins of the comparative sample was used as a driver.

Tester 1 and Tester 2 were prepared from the pretester through PCR using a standard method. Here, Tester 1 was prepared using an SSH forward primer (SSH forward primer_Tester 1, SEQ ID NO: 5) comprising a newly designed adapter 1 and an RS forward primer (underlined portion of SEQ ID NO: 5) and an RS reverse primer, and Tester 2 was prepared using an RS forward primer and an SSH reverse primer (SSH reverse primer_Tester 2, SEQ ID NO: 6) comprising an adapter 2 and an RS reverse primer (underlined portion).
SSH forward primer_Tester 1: 
5′-TCGAGCGGCCGCCCGGGCAGGTCGGAAGCGTGCTGCC-3′ (SEQ
ID NO: 5)
SSH reverse primer_Tester 2:

5′-CAGCGTGGTCGCGGCCGAGGTTCGACCTCTGGGTTATG-3′ (SEQ
ID NO: 6)

1.5 µL of a 30 ng Tester 1, 1.5 µL of a 600 ng driver PCR product, and 1.0 µL of a 4× hybridization solution (300 m MHEPES pH 7.5, 2 M NaCl, 0.8 m MEDTA) were mixed and primary hybridization was performed at a reaction volume of 4.0 µL at 60°C for about 12 hr, and 1.5 µL of a 30 ng Tester 2 (SEQ ID NO: 6), 1.5 µL of a 600 ng driver PCR product, and 1.0 µL of a 4× hybridization solution were mixed, followed by treatment at a reaction volume of 4.0 µL at 95°C for 5 min and then primary hybridization at 60°C for about 12 hr. For the sake of convenience, the former is called "Tester 1 hybrid solution", and the latter is called "Tester 2 hybrid solution".

The Tester 2 hybrid solution was cautiously added to the driver hybrid solution and mixed therewith, and the resulting hybrid solution (a mixed hybrid solution of the Tester 2 hybrid solution and the driver hybrid solution) was then added to the Tester 1 hybrid solution and mixed well using a pipette, followed by secondary hybridization at 60°C for 12 hr. The driver solution was prepared by mixing 1.0 µL of a 130 µg driver, 1.0 µL of a 4× hybridization solution, and 2 µL of water, adding a small amount of mineral oil and performing treatment using a PCR device at 98°C for 90 sec. After termination of the secondary hybridization, the resulting solution was added with Taq polymerase and then subjected to extension at 75°C for 5 min, whereby the cohesive ends of 5' and 3' ends of the double-stranded nucleic acids of the secondary hybridization product were added with bases and thus converted to blunt ends.

After termination of the extension reaction, the resulting solution was subjected to PCR through a standard method using an "adapter 1 primer" (SEQ ID NO: 7) and an "adapter 2 primer" (SEQ ID NO: 8) designed to have the sequence shown below.
Adapter 1 primer:
   5'-TCGAGCGGCCGCCCGGGCAGGT-3' (SEQ ID NO: 7)
Adapter 2 primer:
   5'-CAGCGTGGTCGCGGCCGAGGT-3' (SEQ ID NO: 8)

The PCR product thus obtained was subjected to nested PCR through a standard method using an "RS forward primer" (SEQ ID NO: 3) and an "RS reverse primer" (SEQ ID NO: 4).

The PCR amplification product was subjected to NGS analysis.

### <Example 3-2> Preparation of molecule-binding nucleic acid secondary library using DSN

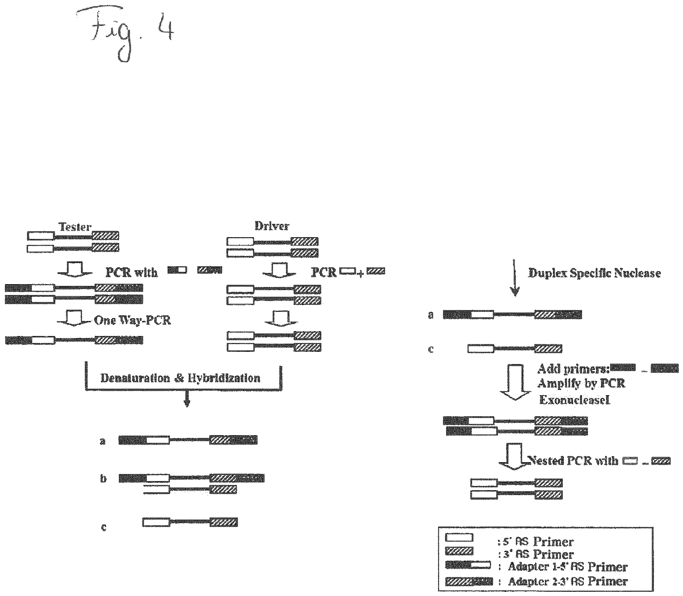

A molecule-binding nucleic acid secondary library was prepared through subtraction using DSN (duplex-specific nuclease) in the steps shown in [FIG. 4].

In order to prepare the molecule-binding nucleic acid secondary library using DSN, the RT-PCR product of the molecule-binding RNA library for the proteins of the analytical sample prepared in <Example 2> was used as a tester, and the RT-PCR product of the molecule-binding RNA library for the proteins of the comparative sample was used as a driver.

PCR was performed using the SSH forward primer_Tester 1 and the SSH reverse primer_Tester 2 as primers from the above tester, after which PCR was performed using the SSH forward primer_Tester 1, thus obtaining tester double-stranded DNA. For the driver, double-stranded DNA was obtained through PCR using the RS forward primer and the RS reverse primer.

The double-stranded DNA thus obtained was subjected to hybridization and then DSN treatment. Particularly, 100 ng/µL double-stranded DNA was prepared, 1.5 µL each of which was then placed in PCR tubes, after which 1 µL, of a 4× hybridization solution and 1.5 µL of distilled water were added thereto and mineral oil was overlaid thereon. Then, heating at 98°C for 3 min and then hybridization at 60°C for 4 hr were performed. After termination of the hybridization, 5 µL of 2× DSN buffer preheated to 60°C (100 mM Tris-HCl pH 8.0, 10 mM MgCl₂, 2 mM dithiothreitol) was added to the reaction mixture. Subsequently, a DSN enzyme (Wako, Japan) at 0.25 Kunitz units was added thereto and the reaction was then carried out. After the reaction for 30 min, 10 µL of 5 mM EDTA was added thereto, thereby terminating the reaction.

60 µL of the resulting reaction product was added with 10 µL of 10× PCR buffer solution, 1 µL of 30 mM dNTP, 1 µL of SSH forward primer_Tester 1 (25 pmol/µL), 1 µL of SSH reverse primer_Tester 2 (25 pmol/µL), 0.5 µL of Taq polymerase (5 µ/µL) and 36.5 µL of tertiary sterile purified water so that the final volume was 100 µL, thus preparing a PCR mixed solution. Using a PCR device, a series of procedures of preliminary denaturation at 95°C for 5 min, denaturation at 95°C for 40 sec, binding at 55°C for 40 sec, and extension at 72°C for 40 sec was repeated 25 times, followed by final extension at 72°C for 5 min. Subsequently, 1 µL of exonuclease I was added thereto and the reaction was carried out for 40 min, thus removing the remaining single-stranded nucleic acids.

The amplification product thus obtained was subjected to nested PCR through a standard method using an "RS forward primer" (SEQ ID NO: 3) and an "RS reverse primer" (SEQ ID NO: 4).

The PCR amplification product was subjected to NGS analysis.

### <Example 3-4> Evaluation of subtraction

In order to evaluate the extent of subtraction of the molecule-binding nucleic acid primary library, the serum sample was immobilized on an NC membrane and then reacted with the molecule-binding nucleic acid secondary library using the subtracted DSN. The molecule-binding nucleic acid binding to the serum protein was separated from the NC membrane and used as a template for RT-PCR, followed by RT-PCR and electrophoresis. The results thereof were confirmed.

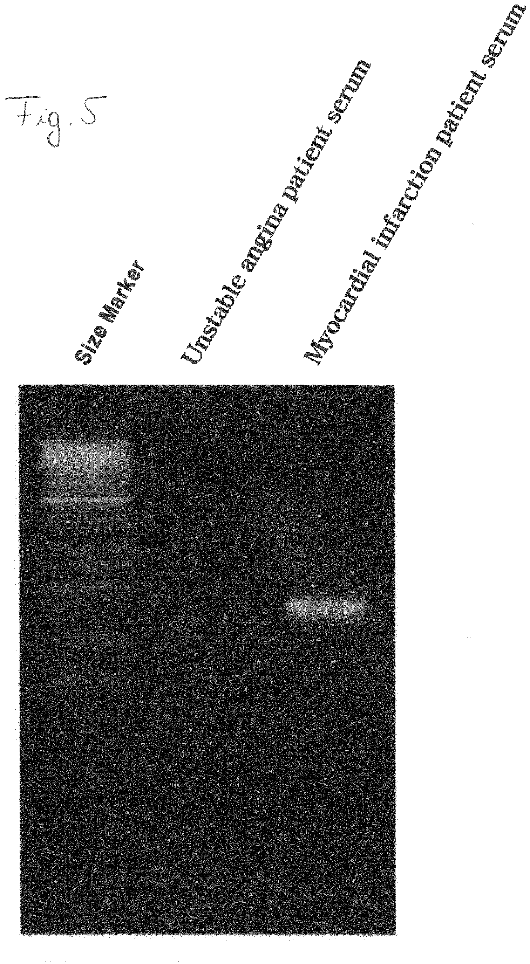

In the present example, the myocardial infarction patient serum was used as an analytical sample and the unstable angina patient serum was used as a comparative sample. The results of evaluation of the molecule-binding nucleic acid library obtained through subtraction are shown in [FIG. 5]. The strong band was formed in the analytical sample and the weak band was formed in the comparative sample, from which the analytical sample was confirmed to be subtracted by the comparative sample.

### <Example 4> Determination of base sequence of molecule-binding nucleic acid library and appearance frequency thereof

The double-stranded DNA pool that was the RT-PCR product of the molecule-binding nucleic acid primary library prepared above and the double-stranded DNA pool that was the RT-PCR product of the molecule-binding nucleic acid secondary library were subjected to NGS (next-generation sequencing), and thus the base sequences of the molecule-binding nucleic acids and the appearance frequencies thereof were determined.

NGS analysis was performed for the DNA pool using a HiSeq 3000 (Illumina).

A sequencing library was prepared using the DNA pool prepared above and a TruSeq DNA sample preparation kit v.2 (Illumina). Particularly, 136 ng DNA was subjected to end repair, adenosine addition, adapter addition and PCR in accordance with the manufacturer's protocol. Washing was performed using Agencourt AMPure XP beads (Beckman-Coulter, USA) included in the TruSeq kit at every step except for the adenosine base addition step. Then, a sequencing library was prepared by performing PCR through 15-cycle reactions using the PCR primers included in the kit in accordance with the manufacturer's protocol.

As the PCR product, the sequencing library was quantified using a Qubit fluorometer (Invitrogen). A 3 ng PCR product per sample was subjected to hybridization at a concentration of 5 pM in a flow cell of a cBOT cluster station of a HiSeq 3000. Bridge amplification on cBOT was performed 28 times per single DNA in a molecule-binding nucleic acid library to form a cluster. Each cluster was linearized and then hybridized with sequencing primers. Such a flow cell was loaded on a HiSeq 3000 and analyzed with a HiSeq 3000 Single-Read 80 Base Pair Recipe capable of sequencing 73 single-read bases and 7 multiplexed bases. Image production and analysis were conducted through Illumina Real-Time Analysis (RTA) and thus base-call files and quality scores were assayed in real time.

After termination of the sequencing of forward and reverse strands, quality analysis was performed using Casava software made by Illumina, and also, downstream analysis was performed using a proprietary software (AptaCDSS, BioEZ) analysis system.

The matching step of the quality analysis was performed for the pattern of 74 base pairs (bp) of the sequence comprising the 5' conserved region (17 bp), variable region (50 bp) and 3' conserved region (17 bp) in <General Formula I> of the oligonucleotides constituting the single-stranded nucleic acid library. The filtering step excludes any sequence that does not match the above pattern. One mismatch was allowed in each conserved region during pattern matching. The conserved region sequence was excluded after filtering, leaving only a 50 bp variable region sequence for downstream analysis. The reverse supplemental tag was coupled with the forward sequence tag. The counting of the molecule-binding nucleic acid reads for each round was performed through round enrichment analysis. The quality analysis and count round results were used to determine the base sequences of certain molecule-binding nucleic acids constituting the library and to determine the appearance frequency thereof.

The sequence analysis was performed on 15,000,000 to 18,000,000 single DNA molecules per DNA pool of molecule-binding nucleic acids.

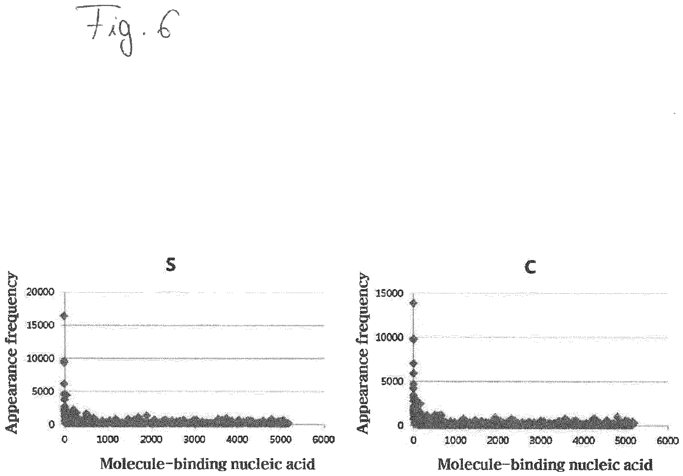

The base sequences and appearance frequencies of 5,395 molecule-binding nucleic acids constituting each of the molecule-binding nucleic acid primary library prepared from the analytical sample S and the molecule-binding nucleic acid primary library prepared from the comparative sample C were compared with each other to confirm that they were different from each other [FIG. 6).

The base sequences of the molecule-binding nucleic acids constituting the molecule-binding nucleic acid secondary library prepared through SSH were subjected to clustering analysis using reference sequences constructed with the molecule-binding nucleic acid sequences determined in the molecule-binding nucleic acid primary library and using Omega Cluster of EBI website (http://www.ebi.ac.uk/Tools/msa/clustalo/).

Through comparison and analysis of these analytical and comparative samples, 1,149 molecule-binding nucleic acids representing the analytical sample were determined.

### <Example 4-2> Determination of biological meaning of molecule-binding nucleic acid

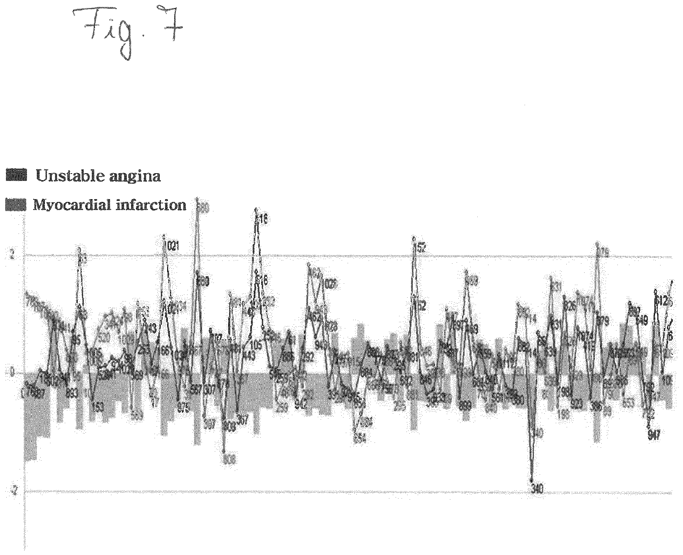

Analysis was performed using the myocardial infarction patient serum (10 cases) as an analytical sample and the unstable angina patient serum (21 cases) as a comparative sample [Table 2]. Reference sequences were constructed with base sequences of 1,149 molecule-binding nucleic acids determined above. Double-stranded DNA, which is the RT-PCR product of the molecule-binding nucleic acid pool separated from the protein-molecule-binding nucleic acid complex pool formed by reacting the sample with a library of 1,149 molecule-binding nucleic acids, was used as a nucleic acid sample to prepare a sequencing library. Then, reads were produced through NGS and compared with the reference sequences, and thus the appearance frequencies of the molecule-binding nucleic acids were analyzed. The distribution of molecule-binding nucleic acids able to distinguish myocardial infarction and unstable angina statistically based on the appearance frequencies of 1,149 molecule-binding nucleic acids using a biological meaning determination system is shown in [FIG. 7].

Based on the analysis results of each of the analytical sample (10 cases) and the comparative sample (10 cases), a database for the analytical sample and the comparative sample was constructed, and molecule-binding nucleic acids that can distinguish the two groups were determined by one-way ANOVA, after which the appearance frequency of the determined molecule-binding nucleic acids was determined by 4 * [(appearance frequency-minimum) / (maximum-minimum)] - 2. The results thereof are shown in [FIG. 7].

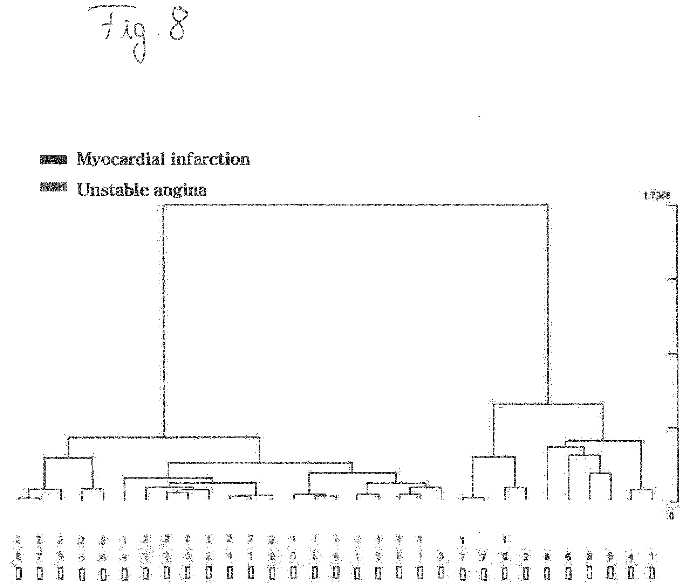

The results of class clustering with the appearance frequency of the selected molecule-binding nucleic acids are shown in [FIG. 8]. As shown in [FIG. 8], the myocardial infarction patient serum and the unstable angina patient serum were able to form independent clusters, from which the patients can be found to be distinguished based on the appearance frequencies of molecule-binding nucleic acids binding to the human serum proteins.

**[Table 2]**

| Clinical information of cardiovascular patients | | |
|---|---|---|
| | Myocardial infarction | Unstable angina |
| Gender | | |
| Male | 10 | 21 |
| Female | 0 | 1 |

| Age | | |
|---|---|---|
| 50-49 | 1 | 0 |
| 60-59 | 5 | 9 |
| 60-69 | 4 | 11 |
| 70-79 | 0 | 1 |
| Total | 10 | 21 |

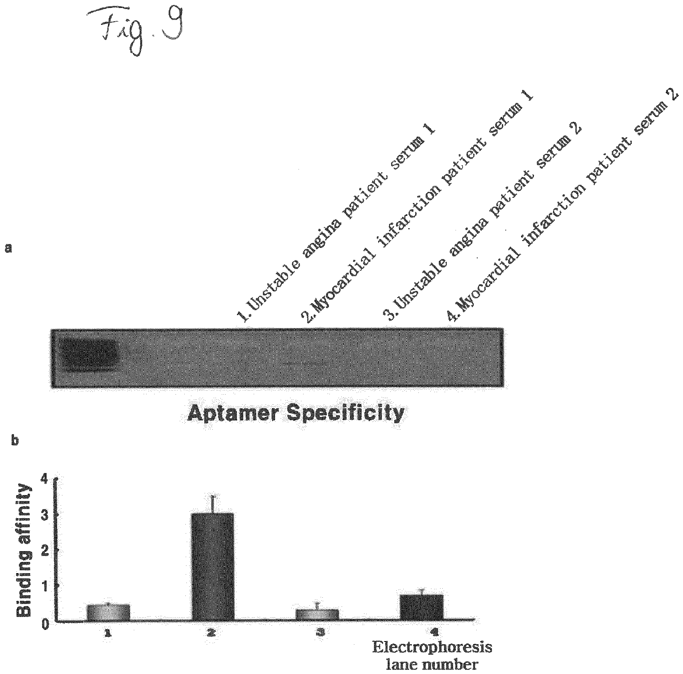

As is apparent from the above results, protein profiles in a certain biosample were searched for and analyzed using molecule-binding nucleic acids binding to multiple proteins of the biosample and the NGS technique, from which molecule-binding nucleic acids specifically binding to the myocardial infarction patient serum protein as the analytical sample and having biological meaning can be selected. Among these, based on the results of evaluation of the extent of binding of serial No. 768 (number assigned to the molecule-binding nucleic acid) molecule-binding nucleic acid to the myocardial infarction patient serum as the analytical sample and to the unstable angina patient serum as the comparative sample, it can be found to specifically respond only to the myocardial infarction patient serum [FIG. 9].

### <Example 4-4> Selection and use of other molecule-binding nucleic acids

### <4-4-1> Molecule-binding nucleic acid for protein in response to anticancer drug

### Selection of molecule-binding nucleic acid

In order to investigate molecule-binding nucleic acids and proteins capable of drug reaction monitoring, total protein of cells treated with the anticancer substance doxorubicin and total protein of untreated cells were prepared as biosamples. The target cancer cell line was liver cancer cells Hep3B, and a liver cancer cell line culture broth was treated with doxorubicin so that the final concentration thereof was 5 µg/mL. 4 hr after treatment, it was confirmed that doxorubicin was absorbed into the cells.

The cells, which were treated with doxorubicin and then cultured for 6 hr (analytical sample), and the untreated cells (comparative sample) were collected and total protein was isolated therefrom. A molecule-binding nucleic acid primary library binding to each cell line was prepared using the single-stranded nucleic acid library prepared in <Example 1>. The library thus prepared was subjected to SSH as in <Example 3> to thus select and attain the liver cancer cell line SSH molecule-binding nucleic acid library as the analytical sample.

### Confirmation of binding capacity of molecule-binding nucleic acid

The molecule-binding nucleic acid pool thus selected and attained was reacted with the cancer cell line Hep3B analytical sample and comparative sample, thus preparing an analytical sample-binding molecule-binding nucleic acid pool and a comparative sample-binding molecule-binding nucleic acid pool, the sequences of which were then determined through NGS and protein profiles were produced.

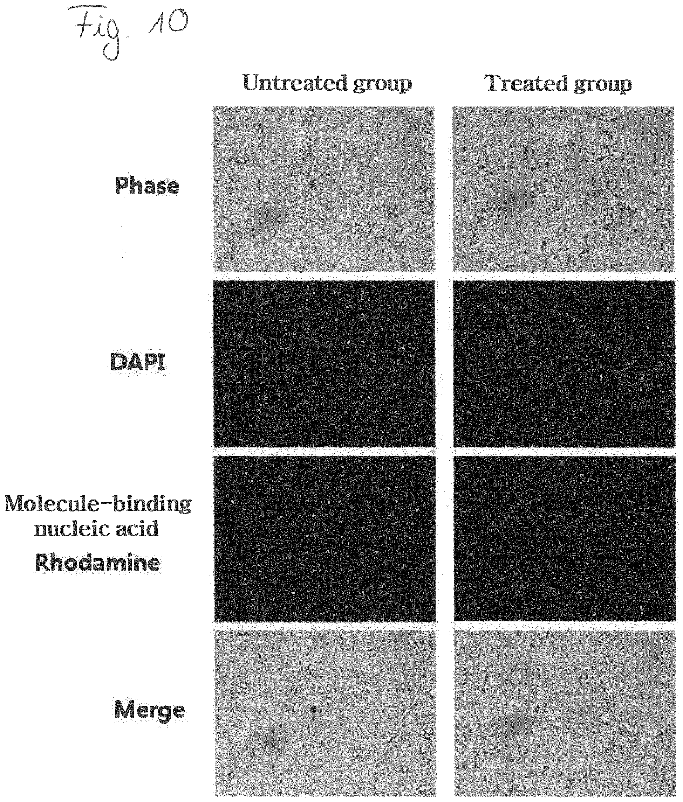

Based on the protein profiles produced and accumulated for the analytical sample and the comparative sample, molecule-binding nucleic acids specifically binding to the analytical sample were selected through ANOVA, and the binding capacity thereof was observed at the cellular level. The selected molecule-binding nucleic acids were labeled with a Rhodamine staining reagent and thus the treated cells and the untreated cells were stained and observed with a fluorescence microscope. The results are shown in [FIG. 10], in which the specific binding of the molecule-binding nucleic acids to the treated cells was confirmed.

### <4-4-2> Liver-cancer-cell-specific molecule-binding nucleic acid

### Selection of molecule-binding nucleic acid

A liver cancer cell line Hep3B sample and a GIBCO hepatocyte sample were treated with the single-stranded nucleic acid library prepared in <Example 1> to thus prepare a molecule-binding nucleic acid primary library binding to each cell line. The library thus prepared was subjected to SSH to thus select and attain a liver cancer cell line SSH molecule-binding nucleic acid library.

The selected molecule-binding nucleic acid pool was reacted with the cancer cell line Hep3B analytical sample and the GIBCO hepatocyte comparative sample, thus preparing a liver cancer cell line Hep3B-binding molecule-binding nucleic acid pool and a GIBCO hepatocyte-binding molecule-binding nucleic acid pool, the cell surface profiles of which were then produced through NGS.

### Confirmation of binding capacity of molecule-binding nucleic acid

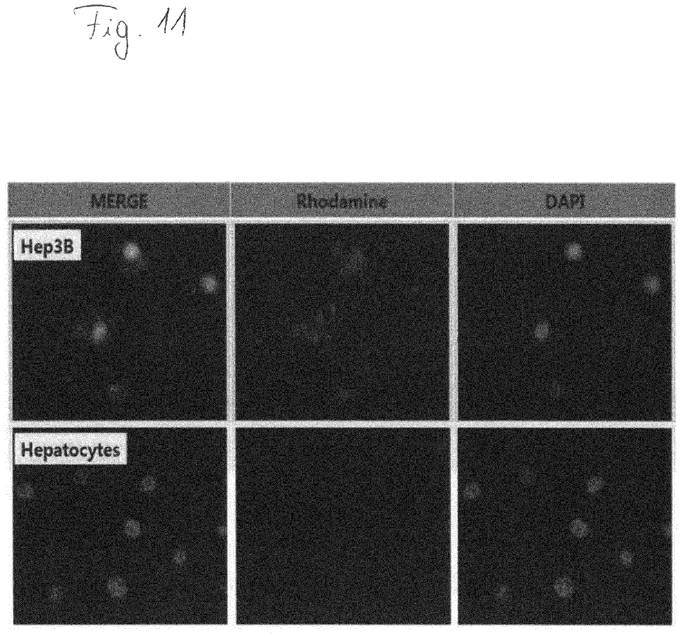

Based on the cell surface profiles produced and accumulated for the cancer cell line Hep3B analytical sample and the GIBCO hepatocyte comparative sample, molecule-binding nucleic acids specifically binding to the liver cancer cell line Hep3B were selected through ANOVA, and the binding capacity thereof was observed at the cellular level [FIG. 11]. The binding capacity of the selected molecule-binding nucleic acids to the liver cancer cell line Hep3B and to the GIBCO hepatocytes was determined by binding certain molecule-binding nucleic acids to the above cells, amplifying the bound molecule-binding nucleic acids and performing electrophoresis, resulting in the finding that they were nucleic acids specifically binding to the liver cancer cell line.

The selected molecule-binding nucleic acids were labeled with a Rhodamine staining reagent, and thus the liver cancer cell line Hep3B and the GIBCO hepatocytes were stained and observed with an optical microscope. The results are shown in [FIG. 11], in which the molecule-binding nucleic acids were specifically bound to the liver cancer cell line Hep3B but scarcely bound to the GIBCO hepatocytes.

### Use of molecule-binding nucleic acid

Next, cdk2 siRNA, inhibiting the function of the cdk2 gene, which is mainly expressed in cancer cells and is thus known as a tumor gene, was prepared. Then, a complex, specifically acting on a liver cancer cell line by binding to a liver-cancer-cell-line-specific molecule-binding nucleic acid, was developed, and the function thereof was evaluated.

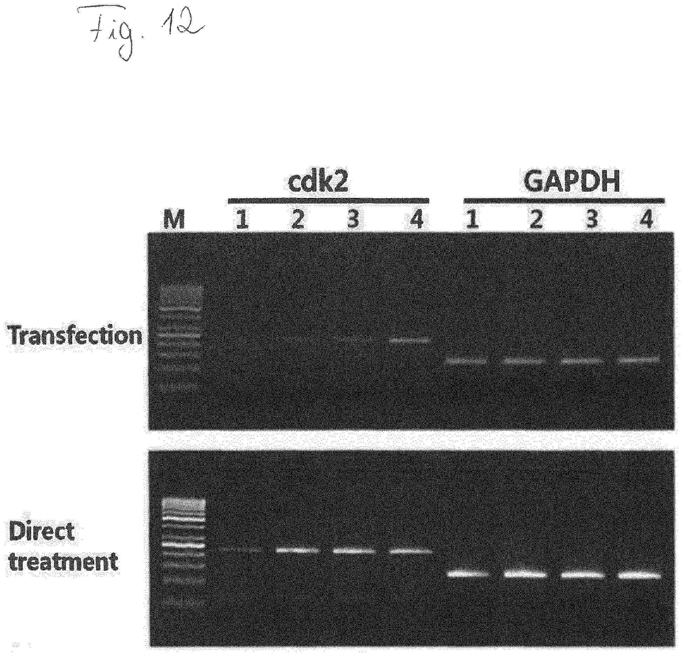

A Hep3B molecule-binding nucleic acid-cdk2 siRNA complex was prepared and then introduced into the cells through transfection or direct treatment. The Hep3B molecule-binding nucleic acid-cdk2 siRNA complex was treated at a concentration of 100 nM or 260 nM, or only cdk2 siRNA was treated for comparison. The liver cancer cell line Hep3B was treated for 3 days, and total RNA was separated from each sample, and thus the expression of cdk2 mRNA was compared and analyzed. The results thereof are shown in [FIG. 12].

The first lane of [FIG. 12] shows the results of treatment of the Hep3B molecule-binding nucleic acid-cdk2 siRNA complex at 100 nM and the second lane shows the results of treatment at 260 nM. The third lane shows the results of treatment of 100 nM cdk2 siRNA and the fourth lane shows the results of the untreated group.

Upon transfection, the amount of cdk2 mRNA was reduced in both the Hep3B molecule-binding nucleic acid-cdk2 siRNA complex and the cdk2 siRNA compared to the untreated group. However, upon direct treatment, the amount of cdk2 mRNA was reduced in the group treated with the Hep3B molecule-binding nucleic acid-cdk2 siRNA complex compared to the untreated group and the group treated with siRNA. In the case of the Hep3B molecule-binding nucleic acid-cdk2 siRNA complex, it can be confirmed that the analytical molecule-binding nucleic acid of the complex was bound to the protein of the liver cancer cell line Hep3B and migrated into the cells and thus acted as siRNA, thereby reducing the amount of cdk2 mRNA.

### <Example 5> Isolation and identification of protein binding to molecule-binding nucleic acid

A database of molecular-binding nucleic acids constructed by disease groups was compared and analyzed to determine useful spots that could contribute to biological meaning analysis, and the corresponding molecule-binding nucleic acids were prepared. Proteins to which the molecule-binding nucleic acids were specifically bound were isolated and identified using the molecule-binding nucleic acids.

Particularly, biotin was attached to one side of the selected molecule-binding nucleic acid (NABM), reacted with streptavidin, and reacted with the serum sample, thus obtaining a serum protein-molecule-binding nucleic acid complex, and the corresponding complex was separated from the sample using the biotin-immobilized support. The band formed through electrophoresis was isolated and the serum protein was identified. Through MALDI-TOF/TOF, the amino acid sequence of the protein binding to the single-stranded nucleic acid was determined. The dissociation constant Kd of the protein identified after isolation from the molecule-binding nucleic acid was measured to be less than 30 × 10⁻⁹.

### <Example 7> Analysis of biosample -simultaneous analysis of nucleic acid and protein

NGS (next-generation sequencing) is a chip-based and PCR-based paired end method in which a whole genome is fragmented and the fragments are hybridized and sequenced at a very high speed. A lot of information about the genome can be produced using NGS.

Using NGS technology, single-nucleotide polymorphism (SNP), which is an allele coding for a genetic trait that appears in 2-5% of the human population, and amino acid mutation resulting from SNP, in which wild-type amino acid is mutated, can be analyzed quickly. WGS (whole genome sequencing) is a method of reading the whole human genome sequence by NGS at several magnifications, such as 10×, 30×, and 50×, WES (whole exome sequencing) is a method of determining the base sequence of only the gene region involved in protein production in the above WGS, and TS (target sequencing) is a technique for sequencing only the gene region involved in target molecule production in the above WGS. Therefore, the data size is generated in descending order of WGS> WES> TS. However, it is advantageous to sequencing a large number of samples when a small region is analyzed. METseq is a sequencing technique for DNA methylation measurement of genes, and RNAseq is a sequencing technique for the expression of genes, namely DNA transcriptome. SV (structural variation) is variation in a large unit (DNA segment) of a chromosome caused by insertion, inversion, translocation, etc. in the mutation, and information thereon can also be produced by NGS.

The process of simultaneously producing protein and nucleic acid information using NGS technology is as follows.

### Construction of Reference Sequence

First, reference sequences should be constructed with base sequences of nucleic acids to be analyzed and base sequences of molecule-binding nucleic acids including aptamers of proteins to be analyzed. In this example, reference sequences were constructed with base sequences of 1,149 molecule-binding nucleic acids and base sequences of the following nucleic acids to be analyzed.

### <Example 7-1> Protein analysis

A sample for protein analysis was prepared from a biosample. The prepared protein sample was brought into contact with a molecule-binding RNA pool, and the formed protein-molecule-binding nucleic acid complex pool was isolated. Particularly, the protein sample was attached to an NC disk and was brought into contact with a molecule-binding nucleic acid pool including aptamers in a reaction solution, and the formed protein-molecule-binding nucleic acid complex pool was washed with a washing solution. Thereby, unbound or nonspecifically bound molecule-binding nucleic acids were removed and the disk was separated. Reverse transcription and PCR were performed on the molecule-binding RNA obtained from the molecule-binding nucleic acid pool binding to multiple proteins attached to the disk, to thus afford a DNA pool. The 136 ng DNA pool thus obtained was subjected to end repair, adenosine addition, adapter addition and PCR using a TruSeq DNA sample preparation kit v.2 (Illumina). Washing was performed with Agencourt AMPure XP beads (Beckman-Coulter) included in the TruSeq kit at every step except for the adenosine base addition step. A sequencing library was constructed by performing PCR through 15-cycle reactions using the PCR primers included in the kit in accordance with the manufacturer's protocol.

### <Example 7-2> DNA analysis

In order to analyze nucleic acid information, a special oligonucleotide design is required to amplify a target gene. The oligonucleotide has the purpose of simultaneously amplifying many targets, and a target-specific sequence (a target hybridization nucleotide sequence) and a 5'-flanking assembly spacer sequence (an overlapping sequence) may be used.

In order to produce multiple target loci assembly sequencing (mTAS) oligonucleotides having optimal lengths that can be annealed at a predetermined temperature, the present inventors used the computer program PrimerPlex2.75 software (PREMIER Biosoft, USA). Oligonucleotide probes were prepared from the target-specific sequence (target hybridization nucleotide sequence) and the 5'-flanking assembly spacer sequence (overlapping sequence). The probes are about 25 bp long and can be annealed at Tm 60°C.

Each target genomic locus is designed to have a gap of 7 bp including the SNP position (i.e. the left of the SNP position: 3 bp; SNP position: 1 bp; and the right of the SNP position: 3 bp). The spacing of the gaps was adjusted to 0-3 bp to facilitate design. Although the assembly spacer sequence was manufactured arbitrarily, the annealing sites present on the assembly sequence were determined based on nearest-neighbor methods (BMC Genomics. 2016; 17: 486.) to calculate temperature values for overlapping regions between oligonucleotides.

A nucleic acid sample was isolated from the biosample using a known method. DNA (gDNA) was extracted from the human serum sample using a QIAamp DNA extraction kit (Qiagen, Germany).

Particularly, for the isolation of gDNA, a serum sample was placed in a 1.5 mL microcentrifuge tube, mixed with 30 µL of protease K and 180 µL of a buffer solution, and reacted at 56°C for 1 hr. The reaction solution thus obtained was purified using a QIAamp spin column and then washed two times with a buffer solution. Next, gDNA was extracted after dissolution in 60 µL of a buffer solution preheated at 70°C, and was then stored at -30°C. Each gDNA extracted above was quantified using a Qubit dsDNA HS Assay Kit and a Qubit 2.0 (Life Technologies, USA).

10 ng gDNA stored at -30°C was used to manufacture a library for NGS using panel primers of 17 target genes. The corresponding panel primers are shown in [Table 3] below.

The library for NGS was prepared using an Ion AmpliSeq Library Kit 2.0 (Life technologies).

Particularly, in order to obtain only the mutation site of the target gene in the DNA extracted from the sample, amplification was performed using the above panel primer pool. 4 µL of 5× HiFi Master Mix, 10 µL of the panel primer pool and 10 ng DNA were mixed and sterile distilled water was added thereto so that the total volume of the reaction solution was 30 µL, thus obtaining a mixed reaction solution. The mixed reaction solution thus prepared was amplified by repeating the procedures at 99°C for 2 min, 99°C for 15 sec and 60°C for 4 min 21 times using a PCR device. The resulting amplification product was added with 2 µL of a Fupa reagent (Thermo Fisher Scientific, USA; used for partial cleavage and phosphorylation), and reacted at 60°C for 10 min, 55°C for 10 min, and 60°C for 30 min, whereby both ends of the amplification product were partially cleaved. The partially cleaved amplification product was added with 2 µL of Ion P1 adapter and 2 µL of Ion Xpress Barcode and reacted at 22°C for 40 min and at 72°C for 10 min, and a sequencing adapter and a barcode capable of distinguishing the samples were bound to the amplification product. The amplification product having the sequencing adapter and the barcode bound thereto was washed with 45 µL of an AMPure XP solution and quantified using an Agilent DNA 1000 chip, thus preparing a sequencing library.

### <Example 7-3> RNA analysis

Serum samples were sampled in an amount of 1.5 ml each and centrifuged at 10,000 g for 5 min at room temperature to recover the cells. Then, total RNA of the recovered cells was extracted using an RNeasy Plus Mini Kit (Qiagen). The concentration and quality of the extracted RNA were measured at 260 nm and 280 nm using a NanoDrop™ 1000 spectrophotometer (NanoDrop Technologies, Wilmington, DE, USA).

The sequence of the cDNA library prepared from the RNA sample was analyzed using a simultaneous mass technique. For example, a cDNA library was synthesized in accordance with the manufacturer's protocol or through slight modification using a product such as mRNA-Seq Sample Preparation Kit (Illumina). 5-fold diluted Klenow DNA polymerase was used in the end-repair step of plasma cDNA. A PCR purification kit (QIAquick MinElute Kit (Qiagen, USA)) was used to purify the end-repaired and adenylated product. A 10-fold diluted paired end adapter was coupled with the plasma cDNA sample, and the adapter-coupled product was purified two times using AMPure XP beads (Agencourt, USA), followed by quantification using an Agilent DNA 1000 chip, thereby preparing a sequencing library.

### <Example 7-4> miRNA analysis

Whole-genome micro RNA (genome-wide miRNA) was analyzed and compared using small ribonucleic acid (small RNA) sequencing in human serum samples. Total RNA rich in small ribonucleic acids was extracted from the serum samples using a mirVAna miRNA isolation kit (Ambion, Austin, TX), and a miRNA library was prepared using an Illumina library preparation protocol (Illumina, San Diego, CA, USA). Each library was indexed with an Illumina adapter (6-base barcode). The small ribonucleic acid (small RNA) library was subjected to size fractionation using a 6% TBE urea polyacrylamide gel, and 150 to 160 base-pair fractions were obtained from the gel and purified. The purified miRNA library was quantified using an Agilent DNA 1000 chip to give a sequencing library.

Although a sequencing library may be prepared by mixing the nucleic acid sample isolated from the fractionated biosample and the protein-molecule-binding nucleic acid complex pool isolated as described above, in this example, sequencing libraries prepared separately were mixed to determine the base sequences. The base sequences of the nucleic acids constituting the prepared sequencing library were compared with the reference sequences to determine the appearance frequency.

In order to determine whether the sequencing library for NGS may be used for sequencing, the length and the amount of the library constructed were measured using an Agilent Bioanalyzer 2100 (Agilent, USA) and a high-sensitivity chip, and libraries were used for sequencing, with lengths ranging from 100 to 400 bp and amounts of 100 pmol/µL or more. Moreover, the libraries were subjected to quality control using a high-sensitivity chip.

As shown above, the PCR product, which was mixed for each sample and to which the barcode sequence of the corresponding sample was attached, was quantified using a Qubit fluorometer (Invitrogen) . A 3 ng PCR product per sample was hybridized at a concentration of 5 pM in a flow cell on a cBOT cluster station of a HiSeq 3000. Bridge amplification using cBOT was performed 28 times per single DNA protein to form a cluster. Each cluster was linearized and hybridized with sequencing primers. The corresponding flow cell was loaded on a HiSeq 3000. This flow cell was analyzed with a HiSeq 3000 Single Read 80 Base Pair Recipe capable of sequencing 73 single-read bases and 7 multiplexed bases. Image production and analysis were performed through Illumina Real-Time Analysis (RTA), and base-call files and quality scores were confirmed in real time.

After termination of the sequencing of forward and reverse strands, quality analysis was performed using Casava software of Illumina, and moreover, downstream analysis was performed using a proprietary software analysis system and reference sequences.

The appearance frequencies of the nucleic acids constituting the determined sequencing library reflect information on nucleic acids and proteins as target molecules, and the biological meaning of the biosample can be determined based on the above analysis results with the biological meaning determination system. The biological meaning of the biological meaning determination system indicates physiological changes of the sample or the person from whom the sample was taken, and also indicates information necessary for the process of making a decision for the purpose of health care such as prevention, diagnosis, treatment, amelioration and therapy based on the clinical test values.

**[Table 3]**

| Target gene and primer | | |
|---|---|---|
| Forward primer | Reverse Primer | Target gene |
| TCCTCATGTACTGGTCCCTCAT | GGTGCACTGTAATAATCCAGACTGT | KRAS |
| CATACGCAGCCTGTACCCA | GTGGATGCAGAAGGCAGACAG | RET |
| TGTCCTCTTCTCCTTCATCGTCT | AGGAGTAGCTGACCGGGAA | RET |
| CCATCTCCTCAGCTGAGATGAC | GGACCCTCACCAGGATCTTG | RET |
| CCTATTATGACTTGTCACAATGTCACCA | TAGACGGGACTCGAGTGATGATT | BRAF |
| CÅCAGCAGGGTCTTCTCTGTTT | CCTTCTGCATGGTATTCTTTCTCTTCC | EGFR |
| TGTCAAGATCACAGATTTTGGGCT | ATGTGTTAAACAATACAGCTAGTGGGAA | EGFR |
| GGTGACCCTTGTCTCTGTGTTC | AGGGACCTTACCTTATACACCGT | EGFR |
| GGAAACTGAATTCAAAAAGATCAAAGTGCT | GGAAATATACAGCTTGCAAGGACTCT | EGFR |
| cttACCAGCTTGTTCATGTCTGGA | AGAGGACTTCGCTGAATTGACC | MET |
| GCAGCGCGTTGACTTATTCATG | CACAGCTACTCTCAGAAAGCACT | MET |
| AACTGAGCTTGTTGGAATAAGGATGTTAT | CCATTTTGGTTTAATGTATGCTCCACAATC | MET |
| CCCATCCAGTGTCTCCAGAAGT | CAAGTGACACTGGTTGTAAATATGCATTT | MET |
| GTTATGACAGGATTTGCACACATAGTT | CCCAAGCCATTCAATGGGATCT | MET |
| CCCTTCTCTTCACAGATCACGA | CAGACAGATCTGTTGAGTCCATGT | MET |
| GCTTGGGCTGCAGACATTTC | GCCAGCTGTTAGAGATTCCTACC | MET |
| TGGTCCTGCACCAGTAATATGC | ATTATAAGGCCTGCTGAAAATGACTGA | KRAS |
| CCATCCACAAAATGGATCCAGACA | GCTCTGATAGGAAAATGAGATCTACTGTTT | BRAF |
| AGGAAATTCCCACTTAGGAACCATTG | AGCAAACTCAGTTGAAATGGTTTGG | MET |
| GTTCAGTGTGTCAAACAGTATTCTTGAATG | GTTGGATGAATTTCATAGACAATGGGATC | MET |
| ACAAGC'ATCTTCAGTTACCGTGAA | AAAACTGCAATTCCTCTTGACTATTCTACA | MET |
| GCTATGGATGTTGCCAAGCTGT | TAACATGAAAAAGGCTTTGGTTTTCAGG | MET |
| GCAACAGCTGAATCTGCAACTC | ATTTTCATTGCCCATTGAGATCATCAC | MET |
| CTGTGTTTAAGCCTTTTGAAAAGCCA | CCAAGTACAACAATTGTATTCACATAGCT | MET |
| CATAATTAAATGTTACGCAGTGCTAACCA | GCAAACCACAAAAGTATACTCCATGGT | MET |
| CAGTCAAACCCTCAGGACAAGA | CCCTCGGTCAGAAATTGGGAAA | MET |
| TCTCAGGAATCACTGACATAGGAGAAG | CGAATGAAATTTCG.AAGATCTCCATGTTT | MET |
| GCTGGTGGTCCTACCATACATG | TTTTTAAAGACTCAGAGCAGGCCTATT | MET |
| GAGGCCAGATGAAATACTTCCTTCA | AAAATCAGCAACCTTGACTGTGAATTTT | MET |
| TGTCCTTTCTGTAGGCTGGATGA | GGTGGTAAACTTTTGAGTTTGCAGA | MET |
| GTGAAGTGGATGGCTTTGGAAAG | AAACTGGAATTGGTGGTGTTGAATTTTT | MET |
| CGTCTCCTGGAGATGGATACTCT | CTGTGGAGGAACTTTTCAAGCTG | FGFR1 |
| GCAGTTACTGGGCTTGTCCAT | GAGGCGGAGAAGCTCTAACAC | FGFR1 |
| GCATGGACAGGTCCAGGTAC | GGAAGACCTGGACCGCATC | FGFR1 |
| CCTTACCTGGTTGGAGGTCAA | GGAGACGTCCCTGACCTTACA | FGFR1 |
| GAGTTCTTTGCTCCACTTGGGA | CCACACTCTGCACCGCTAG | FGFR1 |
| GCACCTTACCTTGTTCAGGCAA | TGGAGTATCCATGGAGATGTGGA | FGFR1 |
| AGGAATGCCTTCAAAAAGTTGGGA | TCCAGTGCATCCATGAACTCTG | FGFR1 |
| GTGATGGCCGAACCAGAAGAAC | CATGTGCCTCTGCCATTGTTG | FGFR1 |
| GAGAGAGGCCTTGGGACTGATA | TCAGAAATGGAGATGATGAAGATGATCG | FGFR1 |
| AGCAGGTTGATGATATTCTTATGCTTCC | CCACTCCCTTAGCCTTTATCCTG | FGFR1 |
| TTAAACCCAATGCCCAGACCCAAA | CCCTTCTTCTTCCCATAGATGCTCT | FGFR1 |
| TCTCCTCTGAAGAGGAGtcatcatc | GGTGTCCGTGTTCATCTGGAAC | FGFR1 |
| GGCAGAAAGAGGACTCCTCAGT | CGACTGCCTGTGAAGTGGATG | FGFR1 |
| TGTAGATCCGGTCAAATAATGCCTC | GGTTTCATCTGAGAAGCAAGGAGT | FGFR1 |
| GCATTAGAGGCCCAGAGAGAGA | TCATCGTCTACAAGATGAAGAGTGGTA | FGFR1 |
| GCTGTGGAAGTCACTCTTCTTGG | CTAACACCCTGTTCGCACTGA | FGFR1 |
| TTGGAATGGGACAAGATTTTCTTTGC | CGAAAGACTGGTCTTAGGCAAAC | FGFR1 |
| AGGACAGAAGCATCACTTACACTTC | CAACTTATGCCACTCTCTGTTTCC | FGFR1 |
| AAATGAAAAGCATGTAATCAGGACTTCCTA | ACACTGCGCTGGTTGAAAAATG | FGFR1 |
| TGTGGTCAGGTTTGAATTCTTTGC | GCCGTAGCTCCATATTGGACATC | FGFR1 |
| CATGCAATTTCTTTTCCATCTTTTCTGG | CTCACAGGTGTTGGGCAGAT | FGFR1 |
| CTTTGTCATTTACAAGTACTTTGCAAACAC | TCCCTAAAGCTGGAGTCCCAAATA | ROS1 |
| TTCTAGTAATTTGGGAATGCCTGGTT | cgcctcTGAATATTTCTTTAATGTTGTCTT | ROS1 |
| GCCTAGGTGCTCCATAATGATGG | AGTCTGGCATAGAAGATTAAAGAATCAAAA | ROS1 |
| ACCAATCATGATGCCGGAGAAAG | ACCTGGTGTGGTTGTCAATACC | ALK |
| TCACCGAATGAGGGTGATGTTTT | GCAGAGCCCTGAGTACAAGC | ALK |
| GGTTGTAGTCGGTCATGATGGT | TGGCCTGTGTAGTGCTTCAAG | ALK |
| ccacttcacctagccAGAATTTTTC | CTGACAGGCGATCTTGAACATCA | EML4 |
| AGATGATAGTATTTCTGCTGCAAGTACTTC | CACATGATCTTCAGAGATTGCAAGAC | EML4 |
| CAAGAAGATGAAATCACTGTGCTAAAGG | CAGCTTCAACTTTCAAAGAAAATATTGCAA | EML4 |
| CTCTGTCGGTCCGCTGAAT | GCTGTGTGCGGAAGGAAAAA | EML4 |
| GTTCTAGTTCAGTCTTCTTCATTGTACCTT | CGGAAGAAGGTAAACATTAGCTCTACAG | EML4 |
| TCTGTTAAGATATGGTTATCGAGGAAAGGA | CAACCATTTCACACAGTCTGTATGG | EML4 |
| AGAGAACTCAGCGACACTACCT | TCTGAGCTTTCCACAAGAAATCACTT | EML4 |
| GCTTCCAAATAGAAGTACAGGTAAGCT | CTGAGCACATGTCAAGAGCAAATC | EML4 |
| TCTTGCCACACATCCCTTCAAA | GCCAGTGTGAGGAGTTTCTGTG | EML4 |
| ATGGTATTTCTTTCTTAGAATGTTAGCCCTA TC | AAATTTACCTTTATTTCTGCTCCTTTTGCTT T | EML4 |
| GAGCATATGCTTACTGTATGGGACT | GCTTTTGCGACTTACGAATAGATAGTTCTA | EML4 |
| TTTTTCATTTGTTTAAATGTGTATTGTTCCA TG | GGAGAAGGTGATGCTCGAATTTG | EML4 |
| gaggaaTCTCATTCTAATGATCAAAGTCCA | CCATTCCCTAGCTCTGTACTTGG | EML4 |
| TGTCAGTTACATGTCTTTGATACTCAGAA | GGGTGTTGAAGGTATTTTCGACAATTTAT | EML4 |
| CTTGGGAAAATTCAGATGATAGCCGTA | CCAGACATACATAACTGTACATGCAAACAT | EML4 |
| CCATAGGGAGACTTTCTCATGTACTC | TAGCATTTAAACATCCCACCACTTCA | EML4 |
| CCCTCCTTCCAAATGGACTTAATTTTAAA | TGCACCACTTCCATTGGTTATACAG | EML4 |
| CCTCGAGCAGTTATTCCCATGTC | AGACAATTCTGCAATGTTAGTTTTTCCC | EML4 |
| aactttacagtttcttgaggtgatttaat gg | TTTCAATTAGCAAAAATTAAACTAAGAAGGG CA | EML4 |
| ACAAAGGTATTCTGTTGTTTCATGTTTCC | CAATGTCTCTAGGTACAGAAGGCAT | EML4 |
| GTTCTACTGAAGTTTTCTTCCAAATAGACAC T | CTCCAGTTAATAACATCCCATTTCTCATCT | EML4 |
| GGCAGTGTGTTCACACTTTGTC | GTGGGACAAAATACCTGAGTTTAGAGTATT | EML4 |
| GACTAAAACTTTGAAGTAGTCATTTTTGTCT T | GAATGAACATGGTAATTGGCCGA | EML4 |
| TGTCTTGTGTTTCAACAGAAGGAGAATAT | GCTGTCATGGTAGAGAAGGATACG | EML4 |
| CCTGAGAAGCTCAAACTGGAGT | cctggccTGATGTTTCCTTTTTAATTTTT | EML4 |
| gcacaccagcgttatgacaaag | TGTGTGGATAGAAACTAGATCTCTGGTT | EML4 |
| GGTGGTTTGTTCTGGATGCAGA | CAATTGCAGTGAAGTGCTGTGAAT | EML4 |
| AGTGGATAGGATTCATTCATTAATTGCCA | TACAGCCAGGAAGGTACCATCT | EML4 |
| ATTTCTCTAGTCAACACTGACCTATTTTATT CT | TCCCAATAATTTTACAACTTGTTCTACTTCA CT | EML4 |
| GTAGCAGTAATTGAATTGATACTTGAAGGAG A | CGCTCCAGGTCCAGAAGAAAATATG | EML4 |
| GCAAATACCATAATTACATGCGGTAAATCT | GCCATGACAACTTGATGCTTATTAAACAAT | EML4 |
| TTTTTAAATGGCATTAGTTCTGTGTGCT | GCTCAAAAGTGCCAAGTCCAATA | EML4 |
| TCTGTTACTCTATCCACACTGCAGAT | ACTTCATGGCCACATAAACACAAAAC | EML4 |
| AACAGTATTGGCTAGCTGTTGAACT | ATACTTACAGTACAATATTTCATAGTCTCCC GA | EML4 |
| CCCAGACAACAAGTATATAATGTCTAACTC | CCCTGACAGACACATCTTAGCatatatata | EML4 |
| CAAGCACTATGATTATACTTCCTGTTTCT | ACAAACCACTTCTTTACATCAGGTGT | EML4 |
| TTAATAAGCATCAAGTTGTCATGGCAAAAA | GGCTCTACAGTAGTTTTGCTCCATA | EML4 |
| AGACTCAGGTGGAGTCATGCTTA | CCTGGTCTAAGAGATGGGACTGA | EML4 |
| ATTTCTGAAACAGGCATGTCAAGAATG | CCAGTTGATATCAGGTGACTGTCATTG | EML4 |
| AGCCATGTCACCAATGTCAGTTTTA | GGCTTTGGTTAGAGTAGTATCCGCTA | EML4 |
| AGTTATCTTTGCCTCAGAATGAGACTG | GTGGGAGAACTGCTTATTCTACTTTCC | EML4 |
| GCCCTTAAATGAGACAGCTGAAGA | GCTTATCTCGTTGCATGGCTCTT | EML4 |
| GAGACCTTGGTGAGCCTCTTTATG | ACATGCAGCTGAAGGAAAAGAGTT | EML4 |
| CAGCTATAAATGCAGGCTTCGAGTA | GTTCCTCGTAAAATAAAGTTTCGTGATGT | EML4 |
| GGAAAGGCAGATCAATTTTTAGTAGGC | ACCCTGAAAATGAAAGACACTCATTGTTAT | EML4 |
| GCTAATTTTTCTGCATCCCTGTGTT | GGGATACTGAAACAGATGGACTTTACAAA | EML4 |
| GTGATAGCTGTTGCCGATGACT | GAGTATCATGGAGAGGAATCAGTAACCTAT | EML4 |
| CTTTTGATGACATTGCATACATTCGAAAGA | CAGTTATCTTTTCAGTTCAATGCATGCT | PIK3CA |
| ATCCGCAAATGACTTGCTATTATTGATG | CCCAGGATTCTTACAGAAAACAAGTG | NRAS |
| GGGTGAGGCAGTCTTTACTCAC | GCCGTTGTACACTCATCTTCCTAG | ALK |
| CCTCACCTCTATGGTGGGATCA | GCTCGCCAATTAACCCTGATTACT | NRAS |
| CAGGTCTCTCCGGAGCAAA | GCCAAGTCCCTGTGTACGA | HER2 |
| AGAACCTCTCAACATTGTCAGTTTTCT | GCTCTGAGTAGAACCATTGCTCA | MET |
| TTGGCACAACAACTGCAGCAAA | CCAGAACATTGTTCGCTGCATTG | ALK |
| GTCTCTCGGAGGAAGGACTTGA | CAGACTCAGCTCAGTTAATTTTGGTTAC | ALK |
| CAGCTGGTGACACAGCTTATG | CTCCGGAGAGACCTGCAAAGAG | HER2 |
| TATGCAGATTGCCAAGGTATGCA | AATGGGAAGCACCCATGTAGAC | HER2 |
| GGGTGTCTCTCTGTGGCTTTAC | ACTCTGTAGGCTGCAGTTCTCA | ALK |
| GCCAATGAAGGTGCCATCATTC | CTCAGGCATCCCAGGCACAT | AKT1 |
| ATTTTACAGAGTAACAGACTAGCTAGAGACA | AAAGAAAAAGAAACAGAGAATCTCCATTTTA GC | PIK3CA |
| GAAATTTAACAGGGTGTTGTTGTGCA | CTGTTCATCTGACAGCTGGGAAT | DDR2 |
| GCTGGAGGAGCTAGAGCTTGAT | GCTTGTGGGAGACCTTGAACAC | MEK1 |
| TGGGTGGTCAGCTGCAAC | CATGCTTCAATTAAAGACACACCTTCTTTAA | ALK |
| GCTCTGAACCTTTCCATCATACTTAGAAAT | ccagactaacaTGACTCTGCCCTATATAAT | ALK |
| AAAGAAGGTGTGTCTTTAATTGAAGCAT | gggtctaatcccatctccagtct | ALK |
| TCAtgttagtctggttcctccaaga | gggttatacttgcaacacagtct | ALK |
| agggaaggctgggtgaacc | actgactttggctccagaacc | ALK |
| ggagcctaaggaagtttcagcaag | cactgctgtgattgcactgaag | ALK |
| ggttctggagccaaagtcagtc | aactataggaaacacaactgaccaagatc | ALK |
| caatcacagcagtggatttgagg | aggcggaattagagcacagatc | ALK |
| GAAGAGCCACATCAtgaaaagatctct | agttaccatccctgcctacaga | ALK |
| ggacctctttggactgcagttt | ggtagagctctttaggatttttcaaaacca | ALK |
| ggttgtcaatgaaatgaattcaccaacata | ACAGAATCTACCCACTGAATCACAATTT | ALK |
| AAACTCCATGGAAGCCAGAACA | ttcattcgatcctcaggtaacccta | ALK |
| tggaccgaccgtgatcagat | ATCTGCCGGTAGAAGGGAGAT | ALK |
| CCTTTGAGGGATGGCACCATAT | GAGACATGCCCAGGACAGATG | ALK |
| CCTTTCCCTCTGCCCTTTTCAA | AGAGAGATAGGAAAATCGGTTTCTGAGTAT | ALK |
| GGCTCACAGGCTGAACAGAAAT | ACTTCTAGCTCCCACATGCTTC | ALK |
| CATTACATAGGGTGGGAGCCAAA | TGTGTATCCTCCTGGCTGATCA | ALK |
| GCTTTCACCATCGTGATGGACA | AAACGGAAGCTCCCAACCTT | ALK |
| CTGATCAGCCAGGAGGATACAC | CCAAGGTGTCACTTCGTTATGC | ALK |
| CCCACCCAATTCCAGGGACTA | GGCTTTCTCCGGCATCATGAT | ALK |
| TGCTTTTCTAACTCTCTTTGACTGCA | GATTGTGGCACAGAGATTCTGATACTT | MET |
| CACAGCCTGAGACACTATTCAGTC | ACTCTCGCTGATCCTCTCTGT | ALK |
| ATGTATTTAACCATGCAGATCCTCAGTTT | ATCTTGTTCTGTTTGTGGAAGAACTCT | PTEN |
| GGATGAGCTACCTGGAGGATGT | CCATCTGCATGGTACTCTGTCT | HER2 |
| TCTTTGTCTTCGTTTATAAGCACTGTCA | GTGTTCTTGCATAAAAACACTTCAAATG | ROS1 |
| TGAAACTTGTTTCTGGTATCCAAAAATCAT | CTGGCTTGCAAAAATCCAGTAGTAG | ROS1 |
| TTCCTTTAGGAAATGTTAACAGTGCATTTG | GATTAAAAATGGTGTAGTATGATTTGTGTAC TT | ROS1 |
| ACTTACCAAAGGTCAGTGGGATTG | CTCTGTGTGCTTAGGTAGAGCTG | ROS1 |
| CTGTGACCACACCTGTCATGTA | AAGAAGGCAAGACCCTTAAAGGAG | RET |
| AGCTCTACCTAAGCACACAGAGTAATA | GTGGTTTGTTgctctctgcaaaaa | ROS1 |
| GGCCCAATGTGTGGATAGAAC | CAGGACAACTTCTCTACATAGCCA | RET |
| GTGTGGATAGAACTTTGGTGGGA | GAAGTGTCCAGGACAACTTCTCT | RET |
| GGGTGGCTATGTAGAGAAGTTGT | CTACATGACAGGTGTGGTCACA | RET |
| CGAAGTACTGAGTCCAAGCCAT | GACAAGTTCCAATGTGCAGAGAAC | RET |
| CTTCTGCACTGAAATCTTTCTACAGAATATA TT | gaatctagataccttgccggtgaag | ROS1 |
| cgactagaagcaactccgttca | gctcactgctcttccttctctct | ROS1 |
| tagattcgctcatcaaaattgactagaagg | gcacagttctttggaaaagcaatttc | ROS1 |
| gggaaattgcttttccaaagaactg | cccagggagttcagtaagcttag | ROS1 |
| atcaaaagcaaggtgtttttgcttt | ATAATGCCAACTATTTAGTATCCAAAGACTG AG | ROS1 |
| AAAAACTAATTAAATCCAGGTAAAAAGCCAT | TCGTTTATGGGTGATTTTGACAAATAAGTTT T | ROS1 |
| GCCATATATAAGTACACAAATCATACTACAC CA | GTTTGTTTTGGTTTATTTTGACTCGTTTATG G | ROS1 |
| TCACCCATAAACGAGTCAAAATAAACCA | CAATGATAAACACTCTTGTACTCTGCaaaa | ROS1 |
| CTGCACATTGGAACTTGTCCATG | TTCTCCTAGAGTTTTTCCAAGAACCAAG | RET |
| GTGGGAATTCCCTCGGAAGAA | TGCCTGGCAGGTACCTTTC | RET |
| AGTCACTGTCCCTGTGACCAT | ACGTAGGGCTATATAATACCAGAAAACTCA | RET |
| GCATAGGGACACGTTTCTGTCAT | AGGCTGTGCTCATTACACCAG | RET |
| CATTTGGAACAGAGGAAAATTTTGACCTC | AGaccactattgccctcttacaga | RET |
| CCAGTGCCAGCTGGTGTAA | GTGGGAAGGCCTGAGAACAC | RET |
| GGGCAGTAAATGGCAGTACC | TCGGCACCACTGGGTACAG | RET |
| TCACATCCAGGTTATATTCCTCAGTAGAAA | gtaagcttcgttccaatactttttctacat | ROS1 |
| TTTAGGACCAAGAAATCTCAGTCTTTGG | GTTTCCTCTACACAACTGAAACTACCT | ROS1 |
| CTCAGTCTTTGGATACTAAATAGTTGGCA | tcctcatgccatagtttgccag | ROS1 |
| GTCCCAACCATGTCAAAATTACAGAC | CCATGGGCTAGACACCACT | HER2 |
| atttgctcttccatgacaggctta | caagtatctcctgatggatgaatgga | BIM |
| ccagtgtgtgtatatcacatacttcattta | agcaattccacttccaagtatatatccaaaa a | BIM |
| AAAGTTAATGTACCGAGGTAAGTTTTCAGT | CTGTAGAATGTCCAGAGAAAATTATGGACT | BIM |
| GAAAGGACTTAGCCAGATGTGAGTTT | CAAAGTCAAGAGAACCACTTATCAACTCA | BIM |
| GTCTTAGTTCATGCCTGAAGACCA | TGACTTCTTTGTGGAAAATGTATTTTGCAA | BIM |
| ATTCTTTACTCAACCCTATCCATGAAGTTC | CTGGCTAAGGCGAACCTCTTTAT | BIM |
| CATTTCTAAATACCATCCAGCTCTGTCT | CATGTGTAGCTGCTGGGATG | BIM |
| GCACACCTGTGAGGTGGTG | CTGAAATGAGTTCACGAGCAGTAGTA | BIM |
| GGGCTGGAAGTTTTATTATTGCTGT | CCTGTTAACTCATTTAGTAAGCAAGGATGT | BIM |
| GTTAACGTCTTCCTTCTCTCTCTGT | TGAGGTTCAGAGCCATGGAC | EGFR |
| CATGCGAAGCCACACTGACGT | CTTTGTGTTCCCGGACATAGTCCA | EGFR |
| TCATCACGCAGCTCATGCCCTT | GTGAGGATCCTGGCTCCTTATCTC | EGFR |
| gctggtACTTTCAGCCTTCACAG | CACCAGCCATCACGTATGCTTC | HER2 |
| TCTCCCATACCCTCTCAGCGTA | CAGCCATAGGGCATAAGCTGTG | HER2 |

As is apparent from [Table 3], the primers were 187 forward primers and 187 reverse primers, and the sequence numbers were designated as SEQ ID NO: 9 to SEQ ID NO: 383 in the order described in Table 3 above.

## Claims

1. A method of quantitatively analyzing a target protein population in a sample to be analyzed, the method comprising:
(a) treating a sample to be analyzed with an aptamer library specific to a target protein population present in the sample so as to form complexes between target proteins and aptamers binding specifically thereto, thereby forming a target protein-aptamer complex population,
(b) isolating the complex population from unbound aptamers, and
(c) analyzing a sequence of each aptamer of the complex population through a next-generation sequencing process so as to quantify each aptamer of the complex population, thereby quantifying each target protein in the complex population.

2. The method of claim 1, wherein the aptamer library is obtained by (i) preparing an aptamer pool having a random sequence to thus have potential binding capacity to various proteins, (ii) reacting the aptamer pool with the target protein population of the same sample as in step (a) so as to induce specific binding between aptamers and target proteins to thereby form a complex population, (iii) isolating the complex population by excluding unbound aptamers, and (iv) amplifying aptamers of the complex population.

3. The method of claim 1, wherein each aptamer of the aptamer library has 5' and 3' regions comprising conserved regions of known sequences and a middle region therebetween comprising a variable region of any random sequence.

4. The method of claim 1, wherein the sample to be analyzed is a processed sample obtained by removing a protein present in a large amount from the sample.

5. The method of claim 1, wherein step (c) is performed by preparing a double-stranded DNA population from aptamers of the complex population and analyzing the double-stranded DNA population through a next-generation sequencing process.

6. The method of claim 5, wherein each aptamer of the aptamer library has 5' and 3' regions comprising conserved regions of known sequences and a middle region therebetween comprising a variable region of any random sequence, whereby the double-stranded DNA population is prepared using a set of a forward primer and a reverse primer.

7. The method of claim 1, wherein the sample to be analyzed before treatment with the aptamer library in step (a) is added with two or more external standard proteins having different quantification values (i.e. concentrations) that are absent in the sample, and the aptamer library in step (a) uses an aptamer library further including aptamers for the external standard proteins, whereby, in step (c), results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins.

8. The method of claim 1, wherein the aptamers are single-stranded DNA or single-stranded RNA.

9. The method of claim 1, wherein the target protein population is a population of unknown proteins, a population of known proteins, or a mixed population of unknown proteins and known proteins.

10. The method of claim 1, wherein when a predetermined protein of the target protein population is an unknown protein, isolating and identifying the unknown protein using an aptamer specific to the unknown protein that is contained in the aptamer library is further performed.

11. The method of claim 1, wherein the quantifying in step (c) is performed by counting a number of reads of the same sequence for the aptamers, counting a number of sequences considered to be the same as the reads taking into account an error frequency of a next-generation sequencing process, and summing the number of reads and the number of sequences so that the target proteins are quantified based on summed values.

12. The method of claim 1, wherein step (c) is performed by comparing a reference sequence, which is a known sequence for each aptamer obtained by analyzing a sequence of each aptamer of the aptamer library, with a sequence analysis result of each aptamer of the complex population.

13. A method of selecting a candidate protein as a biomarker, the method comprising:
(a) treating a sample to be analyzed with an aptamer library specific to a target protein population present in the sample so as to form complexes between target proteins and aptamers binding specifically thereto, thereby forming a target protein-aptamer complex population,
(b) isolating the complex population from unbound aptamers, and
(c) analyzing a sequence of each aptamer of the complex population so as to quantify each aptamer of the complex population, thereby quantifying each target protein in the complex population,
wherein the method further comprises:
(i) performing steps (a) to (c) for an additional sample to be analyzed, which is different from the sample to be analyzed, and
(ii) determining one or more target proteins having different quantification results by comparing target protein quantification results obtained through step (c) between the two samples to be analyzed.

14. The method of claim 13, wherein the aptamer library uses the same aptamer library for the two samples to be analyzed.

15. The method of claim 13, wherein the same aptamer library for the two samples to be analyzed is used, and
the aptamer library is obtained by (i) preparing an aptamer pool having a random sequence to thus have potential binding capacity to various proteins, (ii) reacting the aptamer pool with the target protein population of any one of the two samples to be analyzed so as to induce specific binding between aptamers and target proteins to thereby form a complex population, (iii) isolating the complex population by excluding unbound aptamers, and (iv) amplifying aptamers of the complex population.

16. The method of claim 13, wherein each aptamer of the aptamer library has 5' and 3' regions comprising conserved regions of known sequences and a middle region therebetween comprising a variable region of any random sequence.

17. The method of claim 13, wherein each of the two samples to be analyzed is a processed sample obtained by removing a protein present in a large amount from the sample.

18. The method of claim 13, wherein step (c) is performed by preparing a double-stranded DNA population from aptamers of the complex population and analyzing the double-stranded DNA population through a next-generation sequencing process.

19. The method of claim 18, wherein each aptamer of the aptamer library has 5' and 3' regions comprising conserved regions of known sequences and a middle region therebetween comprising a variable region of any random sequence, whereby the double-stranded DNA population is prepared using a set of a forward primer and a reverse primer.

20. The method of claim 13, wherein the sample to be analyzed before treatment with the aptamer library in step (a) is added with two or more external standard proteins having different quantification values that are absent in the sample, and the aptamer library in step (a) uses an aptamer library further including aptamers for the external standard proteins, whereby, in step (c), results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins,
the additional sample to be analyzed before treatment with the aptamer library in step (i) is added with two or more external standard proteins having different quantification values that are absent in the sample, and the aptamer library uses an aptamer library further including aptamers for the external standard proteins, whereby results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins,
step (ii) is performed by comparing target protein quantification results of the two samples to be analyzed, and
the external standard proteins added to the two samples to be analyzed are the same as each other.

21. The method of claim 13, wherein the aptamers are single-stranded DNA or single-stranded RNA.

22. The method of claim 1, wherein the target protein population is a population of unknown proteins, a population of known proteins, or a mixed population of unknown proteins and known proteins.

23. The method of claim 13, wherein when a predetermined protein of the target protein population is an unknown protein, isolating and identifying the unknown protein using an aptamer specific to the unknown protein that is contained in the aptamer library is further performed.

24. The method of claim 13, wherein the quantifying in step (c) and the quantifying in step (i) are performed by counting a number of reads of the same sequence for the aptamers, counting a number of sequences considered to be the same as the reads taking into account an error frequency of a next-generation sequencing process, and summing the number of reads and the number of sequences so that the target proteins are quantified based on summed values.

25. A method of selecting a candidate protein as a biomarker, the method comprising:
(a) treating each of two samples to be analyzed, including a test sample and a comparative sample, with an aptamer library specific to a target protein population of any one of the two samples to be analyzed so as to form complexes between proteins of a target protein population of each sample and aptamers specifically binding thereto to thereby form a target protein-aptamer complex population in each sample, isolating the complex population from unbound aptamers in each sample, and converting aptamers of the isolated complex population in each sample into a double-stranded DNA population,
(b) removing double-stranded DNA, present in common between double-stranded DNA populations of the test sample and the comparative sample, from the double-stranded DNA population obtained from the test sample, and
(c) analyzing remaining double-stranded DNA of the test sample, from which the double-stranded DNA present in common has been removed, through a next-generation sequencing process to thus analyze each double-stranded DNA sequence of the double-stranded DNA population and determine an abundance of each double-stranded DNA.

26. The method of claim 25, wherein step (c) is performed by amplifying the remaining double-stranded DNA and analyzing a resultant amplification product using a next-generation sequencing process.

27. The method of claim 25, wherein step (b) is performed through an SSH (suppression subtractive hybridization) process or a DSN (duplex-specific nuclease) process.

28. A method of simultaneously analyzing target nucleic acids and target proteins in a sample to be analyzed, suitable for simultaneously performing quantification of target proteins and sequencing and quantification of target nucleic acids, the method comprising:
(a) (i) obtaining a protein sample containing a target protein population from a sample to be analyzed, treating the protein sample thus obtained with an aptamer library specific to the target protein population of the protein sample so as to form complexes between target proteins and aptamers binding specifically thereto to thereby form a target protein-aptamer complex population, isolating the complex population from unbound aptamers, and converting aptamers of the isolated complex population into double-stranded DNA, and (ii) obtaining a nucleic acid sample containing target nucleic acids from a sample the same as the sample to be analyzed and converting the target nucleic acids of the nucleic acid sample into double-stranded DNA fragments,
(b) mixing the double-stranded DNA derived from the aptamers and the double-stranded DNA fragments derived from the target nucleic acids, and
(c) analyzing each double-stranded DNA sequence of a resultant mixture using a next-generation sequencing process, thus obtaining information on each double-stranded DNA sequence and determining an abundance of each double-stranded DNA.

29. The method of claim 28, wherein the target nucleic acids are gDNA, RNA or a mixture thereof.

30. The method of claim 28, wherein the gDNA is gDNA having at least one of sequence deletion, sequence insertion, single-nucleotide polymorphism (SNP), and cytosine methylation.

31. The method of claim 28, wherein the RNA is mRNA, pre-mRNA, ncRNA (noncoding RNA) or a mixture thereof.

32. The method of claim 28, wherein the target proteins are known proteins or unknown proteins, and
the target nucleic acids are known nucleic acids or unknown nucleic acids.

33. The method of claim 28, wherein sequencing libraries are prepared from the double-stranded DNA of the aptamers or the double-stranded DNA fragments of the nucleic acids in step (a), and step (b) is performed by mixing the sequencing libraries.

34. The method of claim 28, wherein the sample to be analyzed before treatment with the aptamer library in step (a) is added with two or more external standard proteins having different quantification values that are absent in the sample, and the aptamer library in step (a) uses an aptamer library further including aptamers for the external standard proteins, whereby, in step (c), results of quantifying the aptamers for the external standard proteins are obtained, in addition to results of quantifying the aptamers for the target proteins, and aptamer quantification results for the external standard proteins and aptamer quantification results for the target proteins are compared, thereby quantifying the target proteins.

35. The method of claim 28, wherein the sample to be analyzed before obtaining the nucleic acid sample in step (a) is added with two or more external standard nucleic acids having different quantification values that are absent in the sample, and in step (c), quantification results for the external standard nucleic acids are obtained, in addition to quantification results for the target nucleic acids, and the quantification results for the external standard nucleic acids and the quantification results for the target nucleic acids are compared, thereby quantifying the target nucleic acids.

36. The method of claim 35, wherein the target nucleic acids are pre mRNA or mRNA.

37. The method of claim 28, wherein the aptamer library is obtained by (i) preparing an aptamer pool having a random sequence to thus have potential binding capacity to various proteins, (ii) reacting the aptamer pool with the target protein population of the same sample as in step (a) so as to induce specific binding between aptamers and target proteins to thereby form a complex population, (iii) isolating the complex population by excluding unbound aptamers, and (iv) amplifying aptamers of the complex population.

38. The method of claim 28, wherein each aptamer of the aptamer library has 5' and 3' regions comprising conserved regions of known sequences and a middle region therebetween comprising a variable region of any random sequence.

39. The method of claim 28, wherein the sample to be analyzed is a processed sample obtained by removing a protein present in a large amount from the sample.

40. The method of claim 28, wherein the nucleic acid sample is a nucleic acid sample having no rRNA.

41. The method of claim 28, wherein each aptamer of the aptamer library has 5' and 3' regions comprising conserved regions of known sequences and a middle region therebetween comprising a variable region of any random sequence, whereby the double-stranded DNA population is prepared using a set of a forward primer and a reverse primer.

42. The method of claim 28, wherein the aptamers are single-stranded DNA or single-stranded RNA.

43. The method of claim 28, wherein the target proteins are unknown proteins, known proteins, or a mixture of unknown proteins and known proteins.

44. The method of claim 28, wherein when a predetermined protein of the target protein population is an unknown protein, isolating and identifying the unknown protein using an aptamer specific to the unknown protein that is contained in the aptamer library is further performed.

45. The method of claim 28, wherein the quantifying in step (c) is performed by counting a number of reads of the same sequence for double-stranded nucleic acids derived from the aptamers or double-stranded nucleic acid fragments derived from the target nucleic acids, counting a number of sequences considered to be the same as the reads taking into account an error frequency of a next-generation sequencing process, and summing the number of reads and the number of sequences so that the target proteins or the target nucleic acids are quantified based on summed values.

46. The method of claim 28, wherein step (c) is performed by comparing a reference sequence, which is a known sequence obtained by analyzing sequences of double-stranded nucleic acids derived from the aptamers or sequences of double-stranded nucleic acid fragments derived from the target nucleic acids, with sequence analysis results of the double-stranded nucleic acids derived from the aptamers or sequence analysis results of the double-stranded nucleic acid fragments derived from the target nucleic acids.
